# EUROPEAN PATENT APPLICATION

(11) **EP 4 809 905 A1**
(43) Date of publication of application: **23.09.2026**
(21) Application number: 24891145.5
(22) Date of filing: 17.10.2024
(51) Int. Cl.: A61B 3/135

(54) **OPHTHALMOLOGICAL DEVICE, METHOD FOR CONTROLLING OPHTHALMOLOGICAL DEVICE, AND RECORDING MEDIUM**

(30) Priority: 16.11.2023 JP 2023195057
(71) Applicant: Topcon Corporation, Tokyo 174-8580 (JP)
(72) Inventor: AZUMA Shinnosuke, Tokyo 174-8580 (JP)
(74) Representative: Gagel, Roland
(86) International application number: PCT/JP2024/036945
(87) International publication number: WO 2025/105103

(57) **Abstract**

An ophthalmological device 1 according to an exemplary embodiment of the present invention executes, by an imaging control unit 71, combined control involving: control of a focal position changing unit 31 for sequentially applying a plurality of different focal positions; control of an exposure amount changing unit 32 for sequentially applying to a plurality of different exposure amounts; and control for causing an imaging optical system 3 to acquire time-series images. Thus, for each of the plurality of different focal positions, a first image group corresponding to a plurality of different exposure amounts is acquired. Furthermore, the ophthalmological device 1 generates, by an image processing unit 81, a single image by combining the plurality of first image groups obtained for the plurality of different focus positions.

## Description

### [TECHNICAL FIELD]

The present disclosure generally relates to an ophthalmic apparatus, a method of controlling an ophthalmic apparatus, and a recording medium.

### [BACKGROUND ART]

Diagnostic imaging serves an important role in the field of ophthalmology. Ophthalmic diagnostic imaging uses various types of ophthalmic apparatuses (ophthalmic imaging apparatuses). Examples of ophthalmic imaging apparatuses include a slit lamp microscope, a fundus camera, a scanning laser ophthalmoscope (SLO), and an optical coherence tomography (OCT) apparatus. An ophthalmic apparatus having an imaging function is not limited to these types of ophthalmic imaging apparatuses. The imaging function may also be provided in ophthalmic examination apparatuses and ophthalmic measurement apparatuses such as refractometers, keratometers, tonometers, specular microscopes, wavefront analyzers, and microperimeters.

One of the most widely and frequently used ophthalmic apparatuses is a slit lamp microscope. The slit lamp microscope is mainly used for anterior segment photography. In addition to cross sectional imaging using slit light illumination, the slit lamp microscope can also be used for imaging performed from a frontal position relative to the anterior segment. Imaging of the anterior segment from the frontal position (front imaging of the anterior segment) can be performed not only by the slit lamp microscope but also by a fundus camera, various types of ophthalmic examination apparatuses, and various types of ophthalmic measurement apparatuses.

### [CITATION LIST]

### [PATENT LITERATURE]

[PATENT DOCUMENT 1] Japanese Unexamined Patent Application Publication No. 2023-3456

### [SUMMARY OF INVENTION]

### [PROBLEM TO BE SOLVED BY INVENTION]

One object of the present disclosure is to achieve an improvement in the quality of an image obtained by front imaging of an anterior segment.

### [SOLUTION TO PROBLEM]

An aspect example of an embodiment is an ophthalmic apparatus comprising: an illumination optical system configured to project illumination light onto an anterior segment of a subject's eye; a photographing optical system configured to perform imaging of the anterior segment, from a front side, onto which the illumination light is projected; a focus position changing unit configured to change a focus position of the photographing optical system; an exposure amount changing unit configured to change an exposure amount for imaging of the anterior segment; an imaging controller configured to perform a combination of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire, for each of the plurality of different focus positions, a first image group corresponding to the plurality of different exposure amounts; and an image processor configured to generate a single image by performing compositing on a plurality of first image groups respectively corresponding to the plurality of different focus positions.

Another aspect example of an embodiment is an ophthalmic apparatus comprising: an illumination optical system configured to project illumination light onto an anterior segment of a subject's eye; a photographing optical system configured to perform imaging of the anterior segment, from a front side, onto which the illumination light is projected; a focus position changing unit configured to change a focus position of the photographing optical system; an exposure amount changing unit configured to change an exposure amount for imaging of the anterior segment; an imaging controller configured to perform a combination of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire a second image group corresponding to a plurality of pairs, each pair including a focus position and an exposure amount and the plurality of pairs including a pair based on a one-to-one correspondence between the plurality of different focus positions and the plurality of different exposure amounts; and an image processor configured to generate a single image by performing compositing on the second image group.

Yet another aspect example of an embodiment is an ophthalmic apparatus comprising: an illumination optical system configured to project illumination light onto an anterior segment of a subject's eye; a photographing optical system configured to perform imaging of the anterior segment, from a front side, onto which the illumination light is projected; a focus position changing unit configured to change a focus position of the photographing optical system; an imaging controller configured to perform a combination of focus position change control that controls the focus position changing unit to sequentially change the focus position of the photographing optical system to a plurality of different focus positions, and imaging control that controls the photographing optical system to acquire time-series images at a constant exposure amount, thereby causing the photographing optical system to acquire a third image group corresponding to the plurality of different focus positions; and an image processor configured to generate a single image by performing compositing on the third image group, wherein the third image group includes only one image for each of the plurality of different focus positions.

Yet another aspect example of an embodiment is an ophthalmic apparatus comprising: an illumination optical system configured to project illumination light onto an anterior segment of a subject's eye; a photographing optical system configured to perform imaging of the anterior segment, from a front side, onto which the illumination light is projected; an exposure amount changing unit configured to change an exposure amount for imaging of the anterior segment; an imaging controller configured to perform a combination of exposure amount change control that controls the exposure amount changing unit to sequentially change the exposure amount for imaging of the anterior segment to a plurality of different exposure amounts, and imaging control that controls the photographing optical system to acquire time-series images at a constant focus position, thereby causing the photographing optical system to acquire a fourth image group corresponding to the plurality of different exposure amounts; and an image processor configured to generate a single image by performing compositing on the fourth image group, wherein the fourth image group includes only one image for each of the plurality of different exposure amounts.

Yet another aspect example of an embodiment is a method for controlling an ophthalmic apparatus configured to image an anterior segment of a subject's eye, the ophthalmic apparatus comprising: an illumination optical system configured to project illumination light onto the anterior segment; a photographing optical system configured to perform imaging of the anterior segment, from a front side, onto which the illumination light is projected; a focus position changing unit configured to change a focus position of the photographing optical system; an exposure amount changing unit configured to change an exposure amount for imaging of the anterior segment; and a processor, the method comprising: an imaging step of causing the processor to perform combination control of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire, for each of the plurality of different focus positions, a first image group corresponding to the plurality of different exposure amounts; and an image processing step of generating a single image by performing compositing on a plurality of first image groups acquired in the imaging step and respectively corresponding to the plurality of different focus positions.

Yet another aspect example of an embodiment is a method for controlling an ophthalmic apparatus configured to image an anterior segment of a subject's eye, the ophthalmic apparatus comprising: an illumination optical system configured to project illumination light onto the anterior segment; a photographing optical system configured to perform imaging of the anterior segment, from a front side, onto which the illumination light is projected; a focus position changing unit configured to change a focus position of the photographing optical system; an exposure amount changing unit configured to change an exposure amount for imaging of the anterior segment; and a processor, the method comprising: an imaging step of causing the processor to perform combination control of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire a second image group corresponding to a plurality of pairs, each pair including a focus position and an exposure amount and the plurality of pairs including a pair based on a one-to-one correspondence between the plurality of different focus positions and the plurality of different exposure amounts; and an image processing step of generating a single image by performing compositing on the second image group acquired in the imaging step.

Yet another aspect example of an embodiment is a method for controlling an ophthalmic apparatus configured to image an anterior segment of a subject's eye, the ophthalmic apparatus comprising: an illumination optical system configured to project illumination light onto the anterior segment; a photographing optical system configured to perform imaging of the anterior segment, from a front side, onto which the illumination light is projected; a focus position changing unit configured to change a focus position of the photographing optical system; and a processor, the method comprising: an imaging step of causing the processor to perform combination control of focus position change control that controls the focus position changing unit to sequentially change the focus position of the photographing optical system to a plurality of different focus positions, and imaging control that controls the photographing optical system to acquire time-series images at a constant exposure amount, thereby causing the photographing optical system to acquire a third image group corresponding to the plurality of different focus positions; and an image processing step of generating a single image by performing compositing on the third image group acquired in the imaging step, wherein the third image group includes only one image for each of the plurality of different focus positions.

Yet another aspect example of an embodiment is a method for controlling an ophthalmic apparatus configured to image an anterior segment of a subject's eye, the ophthalmic apparatus comprising: an illumination optical system configured to project illumination light onto the anterior segment; a photographing optical system configured to perform imaging of the anterior segment, from a front side, onto which the illumination light is projected; an exposure amount changing unit configured to change an exposure amount for imaging of the anterior segment; and a processor, the method comprising: an imaging step of causing the processor to perform combination control of exposure amount change control that controls the exposure amount changing unit to sequentially change the exposure amount for imaging of the anterior segment to a plurality of different exposure amounts, and imaging control that controls the photographing optical system to acquire time-series images at a constant focus position, thereby causing the photographing optical system to acquire a fourth image group corresponding to the plurality of different exposure amounts; and an image processing step of generating a single image by performing compositing on the fourth image group acquired in the imaging step, wherein the fourth image group includes only one image for each of the plurality of different exposure amounts.

Yet another aspect example of an embodiment is a program for causing a computer to perform the method of any of the aspect examples.

Yet another aspect example of an embodiment is a computer-readable non-transitory recording medium storing the program of any of the aspect examples.

### [ADVANTAGEOUS EFFECT OF INVENTION]

Some embodiment examples may be capable of improving the quality of an image obtained by front imaging of an anterior segment.

### [BRIEF DESCRIPTION OF DRAWINGS]

FIG. 1 is a schematic diagram illustrating a configuration of an ophthalmic apparatus according to a non-limiting aspect of an embodiment example.
FIG. 2 is a schematic diagram illustrating a configuration of an ophthalmic apparatus according to a non-limiting aspect of an embodiment example.
FIG. 3A is a schematic diagram illustrating a configuration of an ophthalmic apparatus according to a non-limiting aspect of an embodiment example.
FIG. 3B is a schematic diagram illustrating a configuration of an ophthalmic apparatus according to a non-limiting aspect of an embodiment example.
FIG. 4A is a flowchart illustrating an operation performed by an ophthalmic apparatus according to a non-limiting aspect of an embodiment example.
FIG. 4B is a schematic diagram illustrating an operation performed by an ophthalmic apparatus according to a non-limiting aspect of an embodiment example.
FIG. 5A is a schematic diagram illustrating a configuration of an ophthalmic apparatus according to a non-limiting aspect of an embodiment example.
FIG. 5B is a flowchart illustrating an operation performed by an ophthalmic apparatus according to a non-limiting aspect of an embodiment example.
FIG. 5C is a schematic diagram illustrating a configuration of an ophthalmic apparatus according to a non-limiting aspect of an embodiment example.
FIG. 6A is a schematic diagram illustrating a configuration of an ophthalmic apparatus according to a non-limiting aspect of an embodiment example.
FIG. 6B is a flowchart illustrating an operation performed by an ophthalmic apparatus according to a non-limiting aspect of an embodiment example.
FIG. 6C is a schematic diagram illustrating an operation performed by an ophthalmic apparatus according to a non-limiting aspect of an embodiment example.
FIG. 7A is a flowchart illustrating an operation performed by an ophthalmic apparatus according to a non-limiting aspect of an embodiment example.
FIG. 7B is a schematic diagram illustrating an operation performed by an ophthalmic apparatus according to a non-limiting aspect of an embodiment example.
FIG. 8 is a schematic diagram illustrating an operation performed by an ophthalmic apparatus according to a non-limiting aspect of an embodiment example.
FIG. 9 is a schematic diagram illustrating a configuration of an ophthalmic apparatus according to a non-limiting aspect of an embodiment example.
FIG. 10A is a schematic diagram illustrating a configuration of an ophthalmic apparatus according to a non-limiting aspect of an embodiment example.
FIG. 10B is a schematic diagram illustrating an operation performed by an ophthalmic apparatus according to a non-limiting aspect of an embodiment example.
FIG. 11 is a schematic diagram illustrating a configuration of an ophthalmic apparatus according to a non-limiting aspect of an embodiment example.
FIG. 12A is a flowchart illustrating an operation performed by an ophthalmic apparatus according to a non-limiting aspect of an embodiment example.
FIG. 12B is a schematic diagram illustrating an operation performed by an ophthalmic apparatus according to a non-limiting aspect of an embodiment example.
FIG. 13 is a schematic diagram illustrating a configuration of an ophthalmic apparatus according to a non-limiting aspect of an embodiment example.
FIG. 14A is a flowchart illustrating an operation performed by an ophthalmic apparatus according to a non-limiting aspect of an embodiment example.
FIG. 14B is a schematic diagram illustrating an operation performed by an ophthalmic apparatus according to a non-limiting aspect of an embodiment example.

### [DESCRIPTION OF EMBODIMENTS]

Some non-limiting embodiment examples according to the present disclosure will be described.

Freely selected known techniques or technologies may be combined with embodiment examples. For example, any matters or items described in the documents cited in the present disclosure may be combined with any of the aspects of the embodiment examples. In addition, one or more of the following may be combined with any of the aspects of the embodiment examples: freely selected known documents related to the technical field of the present disclosure; freely selected known techniques or technologies in a technical field similar to the technical field of the present disclosure; and freely selected known techniques or technologies in a technical field different from the technical field of the present disclosure.

For instance, any matters or items disclosed in PATENT DOCUMENT 1 (Japanese Unexamined Patent Application Publication No. 2023-3456) may be combined with the present disclosure by reference. More generally, any technical matters or items disclosed by the applicant of the present application related to the techniques or technologies related to the present disclosure (e.g., matters or items disclosed in patent applications, academic papers, or other literature) may be combined with the present disclosure by reference.

Any two or more of the aspects of the embodiment examples may be combined, at least in part.

One or more of the functions of aspects described in the present disclosure can be implemented by using a circuit configuration (circuitry) or a processing circuit configuration (processing circuitry). The circuitry or the processing circuitry includes any one or more of the following, all of which are configured and/or programmed to execute one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuit configuration or circuitry, and any combination of these. A processor is considered to be processing circuitry or circuitry that includes a transistor and/or another circuitry. In the present disclosure, circuitry, a unit, a means, or any terms similar to these is hardware configured to execute one or more functions disclosed herein, or hardware that is programmed to execute one or more functions disclosed herein. The hardware may be any hardware disclosed in the present specification, or alternatively, known hardware that is programmed and/or configured to execute one or more functions described herein. In a case where the hardware is a processor, which may be considered as a certain type of circuitry, then circuitry, a unit, a means, or any terms similar to these is a combination of hardware and software. In this case, the software is used to configure the hardware and/or the processor.

### < Overview of Embodiment Examples >

Some embodiment examples according to the present disclosure may aim to improve the quality of an image obtained by front imaging of an anterior segment. More specifically, some embodiment examples according to the present disclosure may aim to improve the quality of a front image of an anterior segment through achieving both extension of dynamic range and extension of depth of field.

Extension of dynamic range may provide various advantages. In ophthalmic imaging addressed by the present disclosure, the following advantages are particularly notable. In ophthalmic imaging using light, there are cases in which two or more ocular regions (ocular structures) having significantly different optical properties from each other are depicted in a single image. In some examples, there are cases of acquiring a single image depicting both a cornea and a crystalline lens, which have significantly different light reflectance from one another, and cases of acquiring a single image depicting both a cornea and a sclera, which have significantly different light scattering characteristics from one another. With dynamic range of a typical image sensor, such two or more ocular regions cannot be represented at respective appropriate brightness levels. Some embodiment examples according to the present disclosure can generate a single anterior segment front image in which such respective ocular regions are represented at appropriate brightness levels by extending the dynamic range.

Extension of depth of field may also provide various advantages. In ophthalmic imaging addressed by the present disclosure, the following advantages are particularly obtained. In some ophthalmic diagnosis, it is preferable to make a comprehensive determination based on conditions of a plurality of ocular regions rather than based on a condition of only one ocular region. In order to achieve this, it is desirable that the plurality of ocular regions be depicted free from blur (in focus). However, it is difficult to clearly depict each of the plurality of ocular regions using any depth of field achievable by a typical optical system. For example, it is difficult to acquire a single image in which both a cornea located at a shallow depth position and a crystalline lens located at a deep depth position are in focus. Some embodiment examples according to the present disclosure are configured to extend the depth of field to generate a single anterior segment front image depicting the ocular regions in high detail.

As described above, some embodiment examples according to the present disclosure combine dynamic range extension and depth-of-field extension to generate a single front image of the anterior segment in which a plurality of regions (structures) of the anterior segment are each depicted with appropriate brightness and in focus.

Advantageous effects of the embodiment example according to the present disclosure are not limited to the ability to generate a single image that is excellent in both dynamic range and depth of field. Those skilled in the art will appreciate that each aspect described below provides advantageous effects depending on its features, such as configurations, actions, operations, functions, and applications.

Next, outlines of several aspects of the embodiment example will be described. Exposure amount is related to extension of dynamic range, and focus position is related to extension of the depth of field. Here, the exposure amount is a physical quantity indicating an amount of light detected by a camera (image sensor), in other words, a physical quantity indicating brightness of an image obtained by the camera. The exposure amount is determined by an imaging condition such as brightness of illumination, an F-number of a lens, and a shutter speed.

In the first and second aspects of the embodiment example, a plurality of imaging operations are performed while changing both the exposure amount and the focus position, and a single image is generated from an image group obtained by the plurality of imaging operations. In the third aspect, a plurality of imaging operations are performed at a constant exposure amount (at the same exposure amount) while changing the focus position, and a single image is generated from an image group obtained by the plurality of imaging operations. In the fourth aspect, a plurality of imaging operations are performed at a constant focus position (at the same focus position) while changing the exposure amount, and a single image is generated from an image group obtained by the plurality of imaging operations. Outlines of these four aspects will be described below in more detail.

An overview and several non-limiting features of the first aspect of the embodiment example will now be described. An ophthalmic apparatus according to the present aspect includes an illumination optical system, a photographing optical system, a focus position changing unit, an exposure amount changing unit, an imaging controller, and an image processor.

The illumination optical system is configured to project illumination light onto an anterior segment of a subject's eye. The photographing optical system is configured to perform imaging of the anterior segment, from a frontal position relative to the subject's eye, onto which the illumination light is projected. The illumination optical system and the photographing optical system may be configured in a manner similar to those of a freely selected type of ophthalmic apparatus.

Imaging performed from the front side by the photographing optical system is an operation of imaging the anterior segment from a position in front of the subject's eye. In some examples, the imaging from the front side (front imaging) may be performed in a state in which the optical axis of the photographing optical system is substantially aligned with the axis of the subject's eye. In some other examples, the front imaging may be performed in a state in which the magnitude of deviation (displacement) of the optical axis of the photographing optical system from the axis of the subject's eye is within an allowable range. The allowable range may be freely selected or determined, and examples thereof may include any one or more of the following: a range determined based on an allowable alignment error; a range determined in relation to another imaging mode such as a range determined in relation to a slit photography mode (cross-sectional photography mode) of a slit lamp microscope; and a range determined based on the positions of a plurality of ocular regions to be imaged.

The focus position changing unit is configured to change the focus position of the photographing optical system. The focus position changing unit may include freely selected or designed known configuration and may include a focus lens.

The exposure amount changing unit is configured to change the exposure amount for imaging of the anterior segment. The exposure amount changing unit may include freely selected or designed known configuration.

In some examples, the exposure amount changing unit is configured to change the exposure amount by varying the length of time during which a camera (image sensor) of the photographing optical system detects light, that is, by varying the length of exposure time. The exposure amount changing unit thus configured includes an exposure time changing unit that has a configuration for changing the exposure time of the photographing optical system. The exposure time changing unit may include, for example, at least one of a mechanically controlled shutter (mechanical shutter), an electronically controlled shutter (electronic shutter), a hybrid shutter combining mechanical control and electronic control, and a light amount adjustment filter.

In some examples, the exposure amount changing unit is configured to change the exposure amount by varying the intensity of the illumination light projected onto the anterior segment by the illumination optical system. The exposure amount changing unit thus configured includes an illumination intensity changing unit that has a configuration for changing the intensity of the illumination light. The illumination intensity changing unit may include, for example, at least one of a driving circuit of an illumination light source and a light amount adjustment filter.

The imaging controller is configured to be capable of performing focus position change control, exposure amount change control, and imaging control.

The focus position change control is processing of controlling the focus position changing unit to sequentially change the focus position of the photographing optical system to a plurality of different focus positions.

The exposure amount change control is processing of controlling the exposure amount changing unit to sequentially change the exposure amount for imaging of the anterior segment to a plurality of different exposure amounts. In some examples, the imaging controller is configured to perform, in the exposure amount change control, exposure time change control for controlling the exposure time changing unit to sequentially change the exposure time of the photographing optical system to a plurality of different exposure times. In some examples, the imaging controller is configured to perform, in the exposure amount change control, illumination intensity change control for controlling the illumination intensity changing unit to sequentially change the intensity of the illumination light projected onto the anterior segment to a plurality of different intensities.

The imaging control is processing of controlling the photographing optical system to acquire time-series images of the anterior segment. The time-series images acquired under the imaging control is a plurality of images respectively acquired at a plurality of different time points. Examples of the time-series images include a moving image, a video, a time-lapse image, and a slideshow image.

The imaging controller is configured to combine the focus position change control, the exposure amount change control, and the imaging control to cause the photographing optical system to acquire, for each of the plurality of different focus positions, a plurality of images (first image group) corresponding to that focus position and corresponding to the plurality of different exposure amounts. Through this combination control, a plurality of first image groups respectively corresponding to the plurality of different focus positions are acquired.

Let the plurality of different focus positions be z1, z2, ..., zM (M in number) and let the plurality of different exposure amounts be e1, e2, ..., eN (N in number). Then, the first image group corresponding to each focus position zm (where m = 1, 2, ..., M) includes an image corresponding to the exposure amount e1, an image corresponding to the exposure amount e2, ..., and an image corresponding to the exposure amount eN. In the present example, each first image group includes N images, and the entirety of the M first image groups includes M × N images. Here, M is an integer greater than or equal to two, and N is an integer greater than or equal to two.

The image processor is configured to generate a single image (a single anterior segment front image) by performing compositing on the plurality of first image groups respectively corresponding to the plurality of different focus positions.

The first aspect configured as described above may be capable of extending the dynamic range based on the first image group corresponding to each focus position (each depth position) and corresponding to the plurality of different exposure amounts, and to extend the depth of field based on the plurality of first image groups respectively corresponding to the plurality of different focus positions (the plurality of different depth positions). Accordingly, the first aspect is capable of generating a single anterior segment front image in which a plurality of regions of the anterior segment are each depicted with appropriate brightnesses and in focus. Therefore, the first aspect is capable of improving the quality of an image obtained by front imaging of the anterior segment.

In the first aspect, the image processor may be configured to perform processing of generating a plurality of high dynamic range images respectively corresponding to the plurality of different focus positions by applying high dynamic range compositing to each of the plurality of first image groups, and to perform processing of generating a single image by applying focus compositing (depth compositing) to the plurality of high dynamic range images generated. That is, the image processor may be configured to generate a single anterior segment front image by combining high dynamic range compositing corresponding to each focus position and focus compositing corresponding to the plurality of different focus positions. The present example provides one example of processing performed by the image processor in the first aspect. A high dynamic range image is an image having a dynamic range wider than the dynamic range of the original image, and is also referred to as a dynamic-range-extended image.

Another example of processing performed by the image processor in the first aspect will be described. The present example uses machine learning. The image processor of the present example includes a pre-trained machine learning model. Generation of the machine learning model is performed by means of, for example, training data (teacher data, learning data) generated by imaging of one or more objects (e.g., living eye, extracted eye, model eye, or another object). The training data includes, for example, a plurality of pairs each including an image group (training image group) collected through a plurality of imaging operations performed while combining a plurality of different focus positions with a plurality of different exposure amounts, and a composite image of the training image group (training composite image). The machine learning of the present example is performed by training a neural network including a convolutional neural network (CNN) using the training data. As a result, a machine learning model is constructed that operates to receive, as input, a plurality of anterior segment front images corresponding to combinations of the plurality of different focus positions and the plurality of different exposure amounts, and to output a composite image of the plurality of anterior segment front images. The composite image is a single anterior segment front image.

The configuration and construction method of the machine learning model are not limited to those of the present example. A machine learning model applicable to the image processor of the first aspect may have a freely selected or designed configuration and may be constructed by any method and technique, as long as the model functions to apply image processing that is a combination of high dynamic range compositing or similar processing and focus compositing or similar processing to a plurality of images corresponding to combinations of a plurality of different focus positions and a plurality of different exposure amounts.

In the first aspect, the image processor may include an image selecting processor and an image compositing processor. The image selecting processor is configured to select one or more images from each of the plurality of first image groups acquired by the photographing optical system under combination control performed by the imaging controller. As a result of this, a plurality of images is selected from the plurality of first image groups. The plurality of selected images is referred to as a selected image group. The image compositing processor is configured to generate a single image (a single anterior segment front image) by performing compositing on the selected image group.

The plurality of first image groups acquired by the photographing optical system under the combination control performed by the imaging controller may include images unsuitable for image processing such as image compositing, or images unsuitable for image diagnosis. In some examples, the plurality of first image groups may include images obtained during blinking or images contaminated by unwanted light. Compositing all images in the plurality of first image groups, including such images, may cause deterioration in the quality of the resulting single image.

The present example is configured to, instead of compositing all of the plurality of first image groups, composite a selected image group extracted from the plurality of first image groups. Thus, the present example is capable of more reliably generating a single anterior segment front image in which a plurality of regions of the anterior segment are each depicted with appropriate brightness and in focus. This provides, for example, an advantageous effect that a single anterior segment front image having good quality can be obtained even when the subject's eye is a diseased eye. More generally, it becomes possible to reduce the influence of variations in the condition of the subject's eye and imaging conditions. In other words, robustness can be improved.

In the first aspect, when the image selecting processor selects two or more images from each of one or more first image groups among the plurality of first image groups, that is, when the image selecting processor selects two or more images from any of the first image groups, the image compositing processor may be configured to perform the following processing. The present example provides one example of processing performed by the image processor of the first aspect.

In the present example, for each of the one or more first image groups from which two or more images are selected, the image compositing processor applies high dynamic range compositing to the two or more images selected from the first image group to generate a high dynamic range image. As a result, one or more high dynamic range images respectively corresponding to the one or more first image groups from which the two or more images have been selected are obtained.

Further, the image compositing processor of the present example applies focus compositing to an image group including the one or more high dynamic range images respectively corresponding to the one or more first image groups from which the two or more images have been selected, thereby generating a single image (a single anterior segment front image).

The present example may also be expressed as follows. When the selected image group includes, for each of one or more focus positions among a plurality of different focus positions, two or more images corresponding to the focus position, the image compositing processor of the present example performs the following processing. For each focus position for which two or more images are associated, the image compositing processor applies high dynamic range compositing to the two or more images corresponding to the focus position to generate a high dynamic range image, thereby generating one or more high dynamic range images respectively corresponding to the one or more focus positions. Further, the image compositing processor applies focus compositing to an image group including the one or more high dynamic range images to generate a single image (a single anterior segment front image).

In the first aspect, the image selecting processor may be configured to select two or more images from a first image group corresponding to a focus position located on the focal plane that intersects a plurality of different anterior segment tissues.

In other words, in the present example, when imaging corresponding to a certain focus position yields or is capable of yielding an image in which a plurality of different ocular regions (a plurality of different anterior segment tissues) are in focus, the image selecting processor is configured to select two or more images from the first image group corresponding to this focus position. For example, imaging corresponding to a certain focus position may yield one of the following images: an image in which both the cornea and the crystalline lens are in focus; an image in which both the cornea and the iris are in focus; an image in which both the cornea and the anterior chamber are in focus; or an image in which any three or all of the cornea, iris, crystalline lens, and anterior chamber are in focus. Except in cases where floating material (floating matter) in the aqueous humor is to be observed, the anterior chamber may be excluded from factors used to determine image selection. These anterior segment tissues have respective inherent optical properties (e.g., reflectance and scattering characteristics (scattering intensity), etc.). Accordingly, when imaging is performed at a constant exposure amount (the same exposure amount), these anterior segment tissues are depicted at different brightness levels. According to the present example, two or more images are selected from the first image group corresponding to a focus position in which such a situation occurs or has occurred, and high dynamic range compositing is applied to the selected two or more images to generate a high dynamic range image. In this high dynamic range image, a plurality of different anterior segment tissues are depicted with respective appropriate brightness. The present example selects two or more images from the first image group corresponding to a focus position located on the focal plane that intersects a plurality of different anterior segment tissues and generates one or more high dynamic range images, and further applies focus compositing to an image group including such one or more high dynamic range images to generate a single image (a single anterior segment front image). According to the present example, it is possible to generate a single anterior segment front image in which a plurality of regions of the anterior segment are depicted with respective appropriate brightness and in focus. Additionally, high dynamic range compositing can be omitted for a focus position located on the focal plane that does not intersect a plurality of different anterior segment tissues, thereby reducing processing resources and shortening processing time.

As described in the above example, the image selecting processor selects one or more images from each of the plurality of first image groups. The present example provides one example of the method of determining, from among the plurality of first image groups, a first image group from which two or more images are to be selected.

The first image group from which two or more images are to be selected may be determined in advance, may be determined in parallel with the combination control performed by the imaging controller (in parallel with acquisition of the plurality of first image groups), or may be determined after the plurality of first image groups is acquired. Several examples of the process of determining the first image group from which two or more images are to be selected will be described below.

One example may take the anatomical structure of the eye into consideration. The anatomical structure of the eye may be obtained from a standard eye model or may be obtained from an image acquired by imaging of the anterior segment. Examples of the image of the latter case include a three-dimensional image generated by applying optical coherence tomography scanning (OCT scanning) to the anterior segment, and a three-dimensional image acquired by applying slit scanning using a slit lamp microscope of the Scheimpflug type to the anterior segment.

The present example includes a process of defining a plurality of planes perpendicular to the eye axis (Z-axis), a process of determining the number of anterior segment tissues intersecting each plane (intersection count), and a process of storing information (correspondence information) indicating the correspondence between the Z-coordinate (depth position) of each plane and the intersection count obtained for the plane.

During or after anterior segment imaging, a plane corresponding to each focus position is identified by comparing the coordinate (Z-coordinate) of each focus position applied in the imaging and the Z-coordinates of the plurality of planes. For each of the focus positions, the intersection count corresponding to the plane identified represents the number of anterior segment tissues intersecting the focal plane including the focus position. In a case where the intersection count is two or more, two or more images are selected from the first image group corresponding to the focus position.

Correspondence information for normal eyes (healthy eyes) and correspondence information for diseased eyes (eyes in which the shape or position of an anterior segment tissue has changed) may be prepared and used selectively or in combination. A plurality of pieces of correspondence information for diseased eyes may be prepared. For example, a plurality of pieces of correspondence information corresponding to a plurality of diseases may be prepared, or a plurality of pieces of correspondence information corresponding to a plurality of stages of progression of a single disease may be prepared.

Another example may determine in advance the range of the Z-coordinate in which a plane perpendicular to the eye axis intersects a plurality of different anterior segment tissues based on the anatomical structure of the eye. During or after imaging, it may be determined whether the Z-coordinate of each focus position falls within the predetermined range, thereby determining whether two or more images are to be selected from the first image group corresponding to the focus position.

As described above, the image selecting processor of some examples may include a determining processor configured to determine whether or not a plurality of different anterior segment tissues intersect a focal plane. The determining processor is configured to determine, for each of the plurality of different focus positions, whether a focal plane including the focus position intersects a plurality of different anterior segment tissues by analyzing an image corresponding to the focus position.

In some examples, the determining processor may be configured to apply segmentation to an image included in the first image group corresponding to a certain focus position to divide the image into a plurality of segments (partial regions), and determine whether images of a plurality of different anterior segment tissues are included in the segments. This determination may be performed based at least on any one or more of the following: the Z-coordinate of the focus position; optical properties of the anterior segment tissues; and the anatomical structure of the anterior segment. Through this process, the determining processor determines whether or not the focal plane including the focus position intersects a plurality of different anterior segment tissues.

For each focus position for which the determining processor determines that a corresponding focal plane intersects a plurality of different anterior segment tissues, the image selecting processor selects two or more images from the first image group corresponding to the focus position, and the image compositing processor applies high dynamic range compositing to the two or more images selected from the first image group to generate a high dynamic range image. On the other hand, for each focus position for which the determining processor determines that a corresponding focal plane does not intersect a plurality of different anterior segment tissues, the image selecting processor selects one image from the first image group corresponding to the focus position. The image compositing processor then applies focus compositing to an image group including one or more high dynamic range images generated in the manner described above and one or more images selected in the manner described above, thereby generating a single image (a single anterior segment front image). This series of processing operations is an example of processing performed in a case where both of the following conditions are satisfied: there are one or more focus positions each determined such that a corresponding focal plane intersects a plurality of different anterior segment tissues; and there are one or more focus positions each determined such that a corresponding focal plane does not intersect a plurality of different anterior segment tissues. In the event that all of the plurality of different focus positions falls into the former category, a high dynamic range image corresponding to each focus position may be generated. Conversely, in the event that all of the plurality of different focus positions falls into the latter category, one image may be selected from the first image group corresponding to each focus position.

There may be cases where no image is selected from one or more of the plurality of first image groups respectively corresponding to the plurality of different focus positions. In some examples, all images included in the first image group corresponding to a certain focus position may be unsuitable for image processing or image diagnosis. In such a case, an area corresponding to the focus position from which no image has been selected may be interpolated. The area to be interpolated may be an area that is in focus in imaging at the focus position (an area corresponding to the depth of field). The interpolation processing may be performed, for example, based on one or two first image groups respectively corresponding to one or two focus positions adjacent to the focus position from which no image has been selected. Instead of or in addition to the interpolation processing, a notification may be provided to cause the user to perform imaging again at the focus position, or imaging at the focus position may be performed again automatically. Further, when interpolation processing is performed, additional information (metadata) indicating that the interpolation processing has been performed or additional information indicating an area in which the interpolation has been performed may be generated. The additional information generated may be attached to a freely selected or determined image (e.g., a single anterior segment front image).

In a case where two or more images are selected from a single first image group, the number of images to be selected may be determined in advance or may be determined by processing. The number of images to be selected may be determined based on the Z-coordinate of the focus position or the anatomical structure of the anterior segment. The number of images to be selected may be determined based on the number of types of anterior segment tissues intersecting the corresponding focal plane.

An overview and several non-limiting features of the second aspect of the embodiment example will now be described. As in the first aspect, an ophthalmic apparatus according to the present aspect includes an illumination optical system, a photographing optical system, a focus position changing unit, an exposure amount changing unit, an imaging controller, and an image processor. Among these elements, the elements other than the imaging controller may be the same as those in the first aspect.

The imaging controller of the present aspect is configured, as in the first aspect, to be capable of performing focus position change control, exposure amount change control, and imaging control. By combining the focus position change control, the exposure amount change control, and the imaging control, the imaging controller is configured to cause the photographing optical system to acquire a second image group corresponding to a plurality of pairs, each pair including a focus position and an exposure amount. The plurality of pairs includes a pair formed based on a one-to-one correspondence between the plurality of different focus positions and the plurality of different exposure amounts. The one-to-one correspondence between the plurality of different focus positions and the plurality of different exposure amounts is, in other words, a bijection defined between the set whose elements are the focus positions and the set whose elements are the exposure amounts.

Let the plurality of different focus positions be z1, z2, ..., zM (M in number), let the plurality of different exposure amounts be e1, e2, ..., eM (M in number), and let the plurality of pairs based on the one-to-one correspondence between the plurality of different focus positions and the plurality of different exposure amounts be (z1, e1), (z2, e2), ..., (zM, eM). Then, the second image group includes an image corresponding to the pair (z1, e1), an image corresponding to the pair (z2, e2), ..., and an image corresponding to the pair (zM, eM). The second image group may further include images other than these images corresponding to the pairs (as described later). The second image group of the present aspect includes at least M images.

Note that the second aspect acquires a single image group (the second image group) while the first aspect acquires the plurality of image groups (the plurality of first image groups) respectively corresponding to the plurality of different focus positions.

The image processor is configured to generate a single image (a single anterior segment front image) by performing compositing on the second image group.

The second aspect configured in this manner is capable of extending the dynamic range and extending the depth of field based on the second image group corresponding to the plurality of pairs as described above. Accordingly, a single anterior segment front image can be generated in which a plurality of regions of the anterior segment are each depicted with appropriate brightness and in focus. Therefore, the second aspect makes it possible to improve the quality of an image obtained by front imaging of the anterior segment.

Moreover, the second aspect can improve image quality using a smaller number of images included in the second image group than the number of images included in the first image groups of the first aspect. Therefore, the second aspect further provides advantages such as reduction in the number of imaging operations, shortening of imaging time, reduction in processing resources, and shortening of processing time. It should be noted that the degree of image quality improvement, however, may be greater in the first aspect.

In the second aspect, each pair of a focus position and an exposure amount may be a combination of a focus position corresponding to one anterior segment tissue among a plurality of different anterior segment tissues, and an exposure amount determined in advance based on scattering intensity (magnitude of scattering) of this anterior segment tissue.

Here, the focus position corresponding to an anterior segment tissue may be set, for example, at a freely selected or determined position within the anterior segment tissue or at a freely selected or determined position in the vicinity of the anterior segment tissue. The scattering intensity of an anterior segment tissue may be, for example, a measured value obtained by measurement of a living eye or a statistical value of such measured values, or a measured value obtained by anatomical measurement or a statistical value of such measured values. Determining the exposure amount based on the scattering intensity of the anterior segment tissue may be performed additionally based on freely selected additional information, for example, based on the configuration and/or properties of the illumination optical system, or based on the configuration and/or properties of the photographing optical system.

According to the present example, the plurality of pairs of a focus position and an exposure amount corresponding to scattering characteristics of the plurality of different anterior segment tissues can be prepared as respective imaging conditions. By using these imaging conditions, the second image group can be acquired and a single anterior segment front image can be generated.

Instead of using such default imaging conditions, imaging conditions may be individually determined based on data obtained from the anterior segment of the subject's eye. In some examples, the ophthalmic apparatus according to the second aspect may further include an anterior segment profile acquiring unit configured to acquire a depth profile of the anterior segment of the subject's eye. The anterior segment profile acquiring unit may be configured to generate the depth profile and/or to receive the depth profile from an external source.

The depth profile of the anterior segment indicates a distribution of anterior segment data in the eye axis direction (the Z direction). In some examples, the depth profile may include any one or more of the following: an anterior segment cross-sectional image obtained by imaging of the anterior segment performed with a slit lamp microscope; data generated by applying an OCT scan or an ultrasound scan to the anterior segment (e.g., A-scan data, A-scan image, B-scan image, three-dimensional image, or the like); and axial length data obtained by applying axial length measurement to the subject's eye.

The ophthalmic apparatus of the present example may further include an imaging condition determining processor configured to determine the plurality of pairs of a focus position and an exposure amount based on the depth profile acquired by the anterior segment profile acquiring unit.

The imaging condition determining processor may be configured to identify depth positions of the plurality of different anterior segment tissues based on the depth profile. The depth profile includes data indicating a position of an anterior segment tissue and/or an image of an anterior segment tissue. In some examples, A-scan data of the anterior segment exhibits features such as a peak indicating a position of the cornea (anterior corneal surface, posterior corneal surface) and a peak indicating a position of the crystalline lens (anterior capsule, posterior capsule). An image of the anterior segment includes an image of the cornea, an image of the anterior chamber, an image of the iris, an image of the crystalline lens, or images of other structures. By applying predetermined processing (e.g., signal processing such as peak detection, image processing such as segmentation) to the depth profile, the imaging condition determining processor can identify depth positions (Z-coordinates) of the plurality of different anterior segment tissues.

Furthermore, the imaging condition determining processor may determine the plurality of pairs of a focus position and an exposure amount based on the depth positions of the plurality of different anterior segment tissues identified based on the depth profile and on scattering intensity information of the plurality of different anterior segment tissues. The scattering intensity information may include, for example, standard values of scattering intensity of the anterior segment tissues, or scattering intensities of the anterior segment tissues obtained by a physical technique such as OCT.

According to the present example, the plurality of pairs of a focus position and an exposure amount can be determined as imaging conditions based on data of the anterior segment of the subject's eye. The second image group can be acquired by using the imaging conditions, and a single anterior segment front image can be generated. Accordingly, as compared with the example using default imaging conditions, the present example provides an advantage that imaging can be performed under conditions adapted to an individual subject's eye. On the other hand, the example using default imaging conditions has advantages, as compared with the present example, such as reduced processing resources and shorter processing time.

As described above, the plurality of pairs of a focus position and an exposure amount may include only pairs based on a one-to-one correspondence between a plurality of different focus positions and a plurality of different exposure amounts, or may further include pairs other than those. The latter case will be described below. In the following example, a plurality of images is acquired for a certain focus position.

In the second aspect, the imaging controller may be configured to cause the photographing optical system to acquire, for each of one or more focus positions among the plurality of different focus positions, two or more images respectively corresponding to two or more different exposure amounts. In the present example, the second image group includes two or more images for each of the above one or more focus positions among the plurality of different focus positions, and includes one image for each of another one or more focus positions.

The imaging controller of the present example may be configured to cause the photographing optical system to acquire two or more images respectively corresponding to the two or more different exposure amounts for a focus position located on the focal plane that intersects a plurality of different anterior segment tissues. This configuration may be the same as that in a similar example in the first aspect.

More specifically, the image processor of the present example may include a determining processor. The determining processor may be configured to determine, for each of the plurality of different focus positions, whether a focal plane including the focus position intersects a plurality of different anterior segment tissues. The configuration and operation of the determining processor may be the same as those in a similar example in the first aspect.

The image processor of the present example may be configured to, for each focus position for which two or more images are acquired, apply high dynamic range compositing to the two or more images corresponding to the focus position to generate a high dynamic range image, and to generate a single image (a single anterior segment front image) based on an image group including one or more high dynamic range images generated for one or more focus positions for which two or more images have been acquired. This configuration may be the same as that in a similar example in the first aspect.

The present example is capable of acquiring a high dynamic range image corresponding to a depth position among the plurality of different depth positions. This configuration may improve depiction quality of a plurality of anterior segment tissues having different scattering intensities. In addition, in anterior segment imaging of an individual subject's eye, it becomes possible to automatically determine a depth position at which high dynamic range compositing is to be applied.

As in the similar example described in the first aspect, the following processes may be performed: a process of setting in advance a focus position to which high dynamic range compositing is to be applied (a default focus position); a process of performing imaging at the focus position to acquire two or more images respectively corresponding to two or more different exposure amounts; and a process of generating a high dynamic range image from the two or more images acquired.

In the second aspect, the method of compositing the second image group to generate a single anterior segment front image (the method of image compositing) may be selected or designed as appropriate. In some examples, image compositing may be performed using a machine learning model. The image processor of the present example includes a machine learning model. The machine learning model is trained by machine learning to receive input of a plurality of images having mutually different focus positions and mutually different exposure amounts, and to output a composite image of the plurality of images. The machine learning model is configured to generate a single anterior segment front image from the second image group.

The machine learning model of the present example may have freely selected or designed configuration. In some examples, the machine learning model may include a convolutional neural network constructed by supervised learning using training data prepared in advance. The training data may include, for example, a plurality of pairs, each pair including a training image group having mutually different focus positions and mutually different exposure amounts and a training composite image that is a composite image of the training image group. The machine learning model of the present example may be the same as the machine learning model described in the similar example of the first aspect.

In an example in which machine learning is not used, the image processor may be configured to generate a single image (a single anterior segment front image) by applying image processing including both focus compositing and high dynamic range compositing to a second image group. Details of the present example will be described later.

An overview and several non-limiting features of the third aspect of the embodiment example will now be described. An ophthalmic apparatus according to the present aspect includes an illumination optical system, a photographing optical system, a focus position changing unit, an imaging controller, and an image processor. Among these elements, the elements other than the imaging controller may be the same as those in the first aspect.

Unlike the first aspect (and the second aspect), the ophthalmic apparatus of the present aspect may not include an exposure amount changing unit. However, the ophthalmic apparatus of the present aspect may include an exposure amount changing unit. In this case, control of the exposure amount changing unit is not used in an essential manner in the control for acquiring an image group to which image compositing is to be applied.

The imaging controller of the present aspect is configured to be capable of performing focus position change control similar to that of the first aspect and imaging control different from that of the first aspect. The focus position change control is performed to control the focus position changing unit to sequentially change the focus position of the photographing optical system to a plurality of different focus positions. The imaging control is performed to control the photographing optical system to acquire time-series images at a constant exposure amount. By combining the focus position change control and the imaging control, the imaging controller causes the photographing optical system to acquire a third image group corresponding to the plurality of different focus positions. The third image group includes a plurality of images respectively corresponding to the plurality of different focus positions. The third image group includes only one image for each of the plurality of different focus positions. Let the plurality of different focus positions be z1, z2, ..., zM (M in number). Then, the third image group includes M images.

The image processor is configured to generate a single image (a single anterior segment front image) by performing compositing on the third image group.

According to the third aspect configured as described above, it is possible to extend the dynamic range and to extend the depth of field based on the third image group corresponding to the plurality of different focus positions. Accordingly, a single anterior segment front image can be generated in which a plurality of regions of the anterior segment are depicted with respective appropriate brightness and in focus. Therefore, the third aspect makes it possible to improve the quality of an image obtained by front imaging of the anterior segment.

Moreover, the third aspect is capable of improving image quality using the third image group including a smaller number of images than the first image groups in the first aspect. Therefore, the third aspect further provides advantages such as reduction in the number of imaging operations, shortening of imaging time, reduction in processing resources, and shortening of processing time. Note that the first aspect may exhibit a higher degree of image quality improvement.

Furthermore, since the third aspect is configured to acquire the third image group without performing the exposure amount change control of the first and second aspects, control for acquiring an image group can be simplified.

In the third aspect, the image processor may be configured to generate a (pseudo) high dynamic range image from each of a plurality of images included in the third image group respectively corresponding to the plurality of different focus positions, thereby generating a plurality of (pseudo) high dynamic range images respectively corresponding to the plurality of different focus positions. The image processor may further be configured to generate a single image (a single anterior segment front image) by applying focus compositing to the plurality of high dynamic range images generated.

The processing of generating a pseudo high dynamic range image from a single image may be performed using a freely selected or designed image processing technique. In some examples, the image processor includes a machine learning model. This machine learning model is trained by machine learning to receive input of one image having a first dynamic range and to output an image having a second dynamic range greater than the first dynamic range. By inputting each image of the third image group into this machine learning model, a plurality of high dynamic range images is generated. The machine learning method usable for the present example is not particularly limited and may be selected or designed as appropriate. Examples of the machine learning method include a method based on the number or region of input exposures, a method based on the number of learning tasks, a method based on new sensor data, a method based on a new learning strategy, and a method based on an application.

An overview and several non-limiting features of the fourth aspect of the embodiment example will now be described. An ophthalmic apparatus according to the present aspect includes an illumination optical system, a photographing optical system, an exposure amount changing unit, an imaging controller, and an image processor. Among these elements, the elements other than the imaging controller may be the same as those in the first aspect.

Unlike the first aspect (and the second aspect), the ophthalmic apparatus of the present aspect may not include a focus position changing unit. However, the ophthalmic apparatus of the present aspect may include a focus position changing unit. In this case, control of the focus position changing unit is not used in an essential manner in the control for acquiring an image group to which image compositing is to be applied.

The imaging controller of the present aspect is configured to be capable of performing exposure amount change control similar to that of the first aspect and imaging control different from that of the first aspect. The exposure amount change control is performed to control the exposure amount changing unit to sequentially change the exposure amount for anterior segment imaging to a plurality of different exposure amounts. The imaging control is performed to control the photographing optical system to acquire time-series images at a constant focus position. By combining the exposure amount change control and the imaging control, the imaging controller causes the photographing optical system to acquire a fourth image group corresponding to the plurality of different exposure amounts. The fourth image group includes a plurality of images respectively corresponding to the plurality of different exposure amounts. The fourth image group includes only one image for each of the plurality of different exposure amounts. Let the plurality of different exposure amounts be e1, e2, ..., eN (N in number). Then, the fourth image group includes N images.

The image processor is configured to generate a single image (a single anterior segment front image) by performing compositing on the fourth image group.

According to the fourth aspect configured as described above, it is possible to extend the dynamic range and extend the depth of field based on the fourth image group corresponding to the plurality of different exposure amounts. Accordingly, a single anterior segment front image can be generated in which a plurality of regions of the anterior segment are depicted with respective appropriate brightness and in focus. Therefore, the fourth aspect is capable of improving the quality of an image obtained by front imaging of the anterior segment.

Moreover, the fourth aspect can improve image quality using the fourth image group including a smaller number of images than the first image groups in the first aspect. Therefore, the fourth aspect further provides advantages such as reduction in the number of imaging operations, shortening of imaging time, reduction in processing resources, and shortening of processing time. Note that the degree of image quality improvement may be greater in the first aspect.

Furthermore, the fourth aspect is configured to acquire the fourth image group without performing the focus position change control of the first and second aspects. Accordingly, control for acquiring an image group can be simplified.

In the fourth aspect, the image processor may be configured to generate a (pseudo) depth-of-field-extended image from each of a plurality of images included in the fourth image group respectively corresponding to the plurality of different exposure amounts, thereby generating a plurality of (pseudo) depth-of-field-extended images respectively corresponding to the plurality of different exposure amounts. A depth-of-field-extended image is an image having a depth of field larger than that of the original image and is also referred to as a deepfocus image or a pan-focus image. The image processor may further be configured to generate a single image (a single anterior segment front image) by applying high dynamic range compositing to the plurality of depth-of-field-extended images respectively corresponding to the plurality of different exposure amounts.

The processing of generating a pseudo depth-of-field-extended image from a single image may be performed using a freely selected or designed image processing technique. In some examples, the image processor includes a machine learning model. This machine learning model is trained by machine learning to receive input of one image having a first depth of field and to output an image having a second depth of field larger than the first depth of field. By inputting each image of the fourth image group into this machine learning model, a plurality of depth-of-field-extended images are generated. The machine learning method usable for the present example may be selected or designed as appropriate.

The present disclosure describes several aspects of the embodiment example, including the first to fourth aspects, whose overviews are described above. In addition, the present disclosure describes, in particular, aspects of ophthalmic apparatuses, aspects of methods of controlling the ophthalmic apparatus, aspects of programs, and aspects of recording media. However, the categories of the aspects of the embodiment example are not limited thereto. For example, those skilled in the art will appreciate that the embodiment example according to the present disclosure can provide various aspects of medical methods, various aspects of imaging methods, various aspects of data processing methods, and the like.

### < Ophthalmic Apparatus >

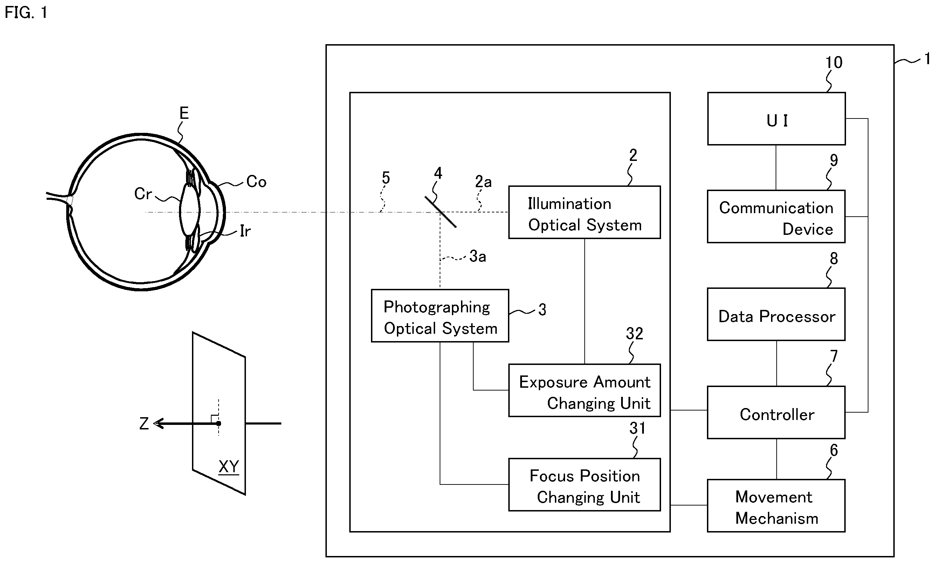

Non-limiting aspects of an ophthalmic apparatus according to the embodiment example will now be described. Examples of the configuration of the ophthalmic apparatus according to the present aspect are shown in FIGs. 1 to 3B.

An ophthalmic apparatus 1 is used for anterior segment imaging of a subject's eye E and includes an illumination optical system 2, a photographing optical system 3, an optical path coupling element 4, a movement mechanism 6, a controller 7, a data processor 8, a communication device 9, a user interface (UI) 10, a focus position changing unit 31, and an exposure amount changing unit 32. The reference sign 5 denotes an optical axis of an optical system of the ophthalmic apparatus 1. The cornea of the subject's eye E is denoted by the reference sign Co, the iris by the reference sign Ir, and the crystalline lens by the reference sign Cr.

The communication device 9 performs data communication between the ophthalmic apparatus 1 and an external apparatus. More specifically, the communication device 9 performs transmission of data to an external apparatus and reception of data transmitted from an external apparatus. The system or method of the data communication employed by the communication device 9 is not particularly limited and may be selected or designed as appropriate. In some examples, the communication device 9 may include one or more communication interfaces selected from various types of communication interfaces, such as a communication interface compliant with the Internet, a communication interface compliant with a dedicated line, a communication interface compliant with a local area network (LAN), and a communication interface compliant with near-field communication. The data communication may include either wireless communication, wired communication, or both.

Data transmitted or received by the communication device 9 may be encrypted data. The controller 7 and/or the data processor 8 may include either or both of an encryptor and a decryptor. The encryptor is configured to encrypt data to be transmitted by the communication device 9. The decryptor is configured to decrypt data having been received by the communication device 9.

The user interface 10 includes a freely selected or designed user interface device, such as a display device and an operation device. A user, such as a physician, a subject, or an assistant, may use the user interface 10 to operate the ophthalmic apparatus 1 and input information into the ophthalmic apparatus 1. At least a part of the user interface 10 may be a peripheral device of the ophthalmic apparatus 1.

The display device displays various types of information under the control of the controller 7. The display device may include a flat panel display such as a liquid crystal display (LCD). The operation device includes a device used for operating the ophthalmic apparatus 1 and a device used for inputting information. The operation device may include, for example, a button, a switch, a lever, a dial, a handle, a knob, a mouse, a keyboard, a trackball, or an operation panel. A device in which the display device and the operation device are integrated, such as a touchscreen, may also be used.

The direction along the axis of the subject's eye E is defined as the Z direction, and the plane perpendicular to the Z direction is defined as the XY plane. The left-right direction (horizontal direction) relative to the subject is defined as the X direction, and the direction perpendicular to both the X direction and the Z direction (vertical direction, body-axis direction) is defined as the Y direction.

The optical path coupling element 4 is configured to couple an optical path of the illumination optical system 2 (illumination optical path) and an optical path of the photographing optical system 3 (photographing optical path). The optical path coupling element 4 may be, for example, a beam splitter such as a half mirror or a dichroic mirror. The optical axis of the optical path extending from the optical path coupling element 4 toward the subject's eye E, that is, the optical axis common to the illumination optical path and the photographing optical path, is an optical axis 5. In this way, the illumination optical system 2 and the photographing optical system 3 are coaxially coupled by the optical path coupling element 4 in the present example.

In FIG. 1, the optical axis 2a of the illumination optical system 2 is arranged parallel to the optical axis 5, and the optical axis 3a of the photographing optical system 3 is arranged perpendicular to the optical axis 5. However, the relative positional relationship between the illumination optical system 2 and the photographing optical system 3 is not limited to this example. In some examples, the optical axis 2a of the illumination optical system 2 may be arranged perpendicular to the optical axis 5, and the optical axis 3a of the photographing optical system 3 may be arranged parallel to the optical axis 5.

One or more elements of the illumination optical system 2 and/or one or more elements of the photographing optical system 3 may be arranged at a position closer to the subject's eye E than the optical path coupling element 4. In some examples, an optical system configuration may be adopted in which the illumination optical system 2 and the photographing optical system 3 share an objective lens arranged at a position closer to the subject's eye E than the optical path coupling element 4.

An optical system configuration may be adopted in which the illumination optical system 2 and the photographing optical system 3 are not coaxially coupled. For example, as in a typical slit lamp microscope, the illumination optical system 2 and the photographing optical system 3 may be configured independently of each other, and the relative position between the illumination optical system 2 and the photographing optical system 3 may be changeable.

In anterior segment imaging, alignment of the ophthalmic apparatus 1 (the illumination optical system 2 and the photographing optical system 3) relative to the subject's eye E may be performed. For example, alignment is performed to adjust the position of the ophthalmic apparatus 1 in such a manner as to coincide the optical axis 5 with the axis of the subject's eye E (XY alignment). Further, alignment is performed to adjust the position of the ophthalmic apparatus 1 so that the distance between the subject's eye E and the ophthalmic apparatus 1 coincides with a predetermined working distance (Z alignment). The aspect of alignment is not limited to the present example. Alignment may be performed automatically or manually.

The illumination optical system 2 has a configuration for projecting illumination light onto the anterior segment of the subject's eye E. The illumination optical system 2 may have a configuration similar to that of an illumination optical system of a known ophthalmic apparatus. Although not illustrated in the drawings, the illumination optical system 2 includes an illumination light source, a lens, and like optical elements.

In a non-limiting example in which the ophthalmic apparatus 1 is a slit lamp microscope, the illumination optical system 2 includes an illumination light source, a positive lens, a slit forming unit, an objective lens, etc. Illumination light output from the illumination light source passes through the positive lens and is projected onto the slit forming unit. Light that has passed through a slit formed by the slit forming unit (slit light) is refracted by the objective lens and is projected onto the anterior segment of the subject's eye E. In a case where the ophthalmic apparatus 1 is a slit lamp microscope, the optical path coupling element 4 is not required. In a case where the ophthalmic apparatus 1 is an anterior segment imaging modality of a different type, a known configuration of the modality may be adopted for the illumination optical system 2.

The photographing optical system 3 has a configuration for imaging of the anterior segment, onto which illumination light is projected by the illumination optical system 2, from a frontal position relative to the subject's eye. The anterior segment front imaging performed by the photographing optical system 3 is carried out, for example, after the above-described XY alignment (and the Z alignment) has been conducted. A front image of the anterior segment is obtained by the anterior segment front imaging. The front image is an image represented in the XY plane, and pixel positions thereof are defined by coordinates in the XY coordinate system.

The photographing optical system 3 may have a configuration similar to that of a photographing optical system of a known ophthalmic apparatus. Although not illustrated in the drawings, the photographing optical system 3 includes a lens, an image sensor, and like elements. The photographing optical system 3 may be configured to function as a video camera capable of capturing moving images.

In addition to imaging from the front side relative to the subject's eye E, the photographing optical system 3 may also be configured to perform imaging from directions other than the front.

The ophthalmic apparatus 1 may be configured to construct a front image from an image other than a front image. In some examples, the photographing optical system 3 may be configured to acquire an image from an oblique position relative to the subject's eye E (an oblique image of the anterior segment), and the data processor 8 may be configured to apply image processing such as projection to the oblique image of the anterior segment to generate an anterior segment front image. In another example, the illumination optical system 2 and the photographing optical system 3 may be configured to acquire a dataset by applying a slit scan using a Scheimpflug optical system or an OCT scan to the anterior segment. In this example, the data processor 8 may be configured to construct a three-dimensional anterior segment image from the dataset and to generate an anterior segment front image from the three-dimensional anterior segment image. In these cases, the anterior segment front image generated by the data processor 8 may be used as an alternative image to the anterior segment front image acquired by the photographing optical system 3 of the present example.

In a non-limiting example in which the ophthalmic apparatus 1 is a slit lamp microscope, the photographing optical system 3 includes an objective lens, a variable magnification optical system, an imaging lens, an image sensor, and like elements. Light that has entered the photographing optical system 3 is refracted by the objective lens and the variable magnification optical system, and then forms an image on a light detecting plane of the image sensor by the imaging lens. The image sensor may be an area sensor having a two-dimensional image capturing area, such as a charge-coupled device (CCD) image sensor or a complementary metal-oxide-semiconductor (CMOS) image sensor. In a case where the ophthalmic apparatus 1 is an anterior segment imaging modality of another type, a known configuration of that modality may be adopted for the photographing optical system 3.

The focus position changing unit 31 has a configuration for changing the focus position of the photographing optical system 3. The configuration of the focus position changing unit 31 that may be employed to move the focus position is not particularly limited and may be selected or designed as appropriate. One or more elements of the focus position changing unit 31 may be provided in the photographing optical system 3.

In some examples, the focus position changing unit 31 may be configured to move the focus position of the photographing optical system 3 by moving at least a part of the photographing optical system 3. In some other examples, the focus position changing unit 31 may be configured to move the focus position of the photographing optical system 3 by changing the focal length of the photographing optical system 3. In still some other examples, the focus position changing unit 31 may be configured to move the focus position of the photographing optical system 3 by adding an optical element (such as a lens) to the photographing optical system 3 or by removing an optical element from the photographing optical system 3.

In one example, the focus position changing unit 31 may be configured to move the focus position by moving the entire photographing optical system 3 in the direction along the photographing optical axis. In another example, the focus position changing unit 31 may be configured to move the focus position by moving the objective lens of the photographing optical system 3 in the direction along the photographing optical axis. In yet another example, the focus position changing unit 31 may be configured to move the focus position by changing the focal length through moving a focusing lens provided between the objective lens and the imaging lens in the direction along the photographing optical axis. In any of these examples, the focus position changing unit 31 includes a mechanism configured to move a movable member.

The exposure amount changing unit 32 has a configuration for changing an exposure amount in anterior segment imaging. The configuration of the exposure amount changing unit 32 that may be employed to change the exposure amount is not particularly limited and may be selected or designed as appropriate. One or more elements of the exposure amount changing unit 32 may be provided in the illumination optical system 2 or the photographing optical system 3.

In some examples, the exposure amount changing unit 32 may be configured to change the exposure amount by using the illumination optical system 2. In some examples, the exposure amount changing unit 32 may be configured to change the exposure amount by using the photographing optical system 3.

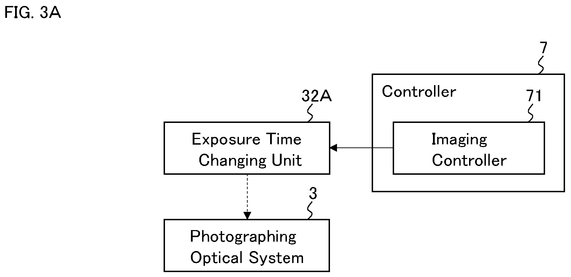

In one example, the exposure amount changing unit 32 may be configured to change the exposure amount by changing the exposure time of the image sensor (camera) of the photographing optical system 3. The exposure amount changing unit 32 of the present example includes an exposure time changing unit 32A having a configuration for changing the exposure time of the photographing optical system 3 (see FIG. 3A). The exposure time changing unit 32A may include, for example, a mechanical shutter or an electronic shutter provided in the photographing optical path. The exposure time changing unit 32A may include an electronically controlled light-amount adjustment filter disposed in the photographing optical path or a light-amount adjustment filter that can be inserted into and removed from the photographing optical path.

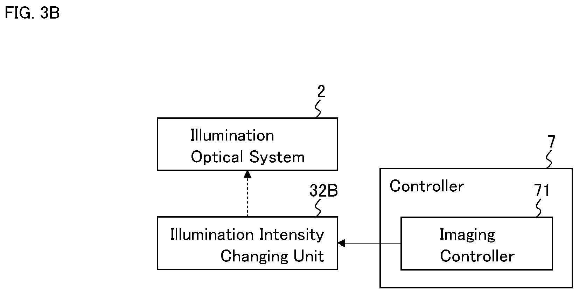

In another example, the exposure amount changing unit 32 may be configured to change the exposure amount by changing the intensity of illumination light projected onto the anterior segment by the illumination optical system 2. The exposure amount changing unit 32 of the present example includes an illumination intensity changing unit 32B having a configuration for changing the intensity of the illumination light (see FIG. 3B). The illumination intensity changing unit 32B may include, for example, any of the following: a light source driving circuit configured to drive a light source of the illumination optical system 2 (illumination light source); an electronically controlled light-amount adjustment filter disposed in the illumination optical path; and a light-amount adjustment filter that can be inserted into and removed from the illumination optical path.

The movement mechanism 6 may be configured to move the illumination optical system 2 and the photographing optical system 3 as a single unit. For example, the movement mechanism 6 may be capable of moving the illumination optical system 2 and the photographing optical system 3 in the X direction, the Y direction, and the Z direction. In cases such as when the ophthalmic apparatus 1 is a slit lamp microscope, the movement mechanism 6 may be capable of moving the illumination optical system 2 and the photographing optical system 3 independently of each other.

The controller 7 is configured to control each component of the ophthalmic apparatus 1. For example, the controller 7 controls elements of the illumination optical system 2 (e.g., the illumination light source, an optical element, and a mechanism), elements of the photographing optical system 3 (e.g., an optical element and a mechanism), the focus position changing unit 31, the exposure amount changing unit 32, the movement mechanism 6, the data processor 8, the communication device 9, and the user interface 10.

The controller 7 includes a processor, a main memory, and an auxiliary storage device. The auxiliary storage device stores computer programs such as various control programs. These computer programs may be stored in a computer or storage device accessible by the ophthalmic apparatus 1. The functions of the controller 7 are implemented by cooperation between software such as the control programs and hardware such as the processor.

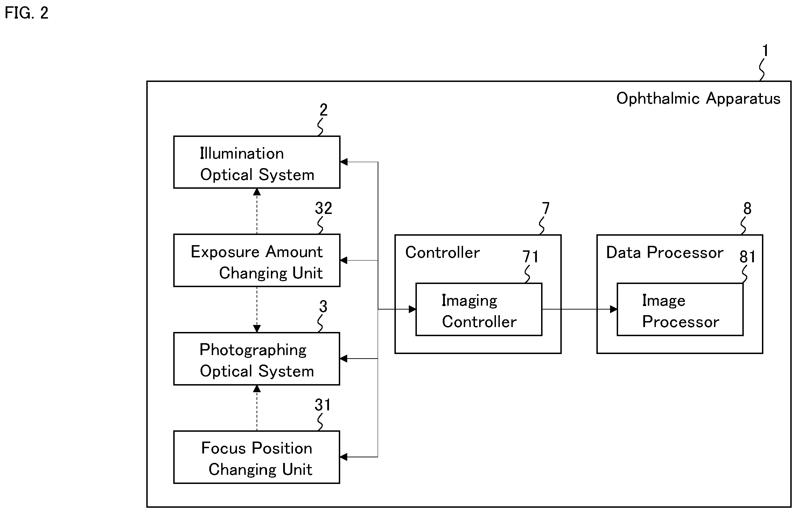

The controller 7 includes an imaging controller 71 (see FIGs. 2, 3A, and 3B). The imaging controller 71 of the present example is configured to perform, in combination, control of the focus position changing unit 31, control of the exposure amount changing unit 32, and control of the photographing optical system 3. The imaging controller 71 performs the control of the focus position changing unit 31 to sequentially change the focus position of the photographing optical system 3 to a plurality of different focus positions (focus position change control). The imaging controller 71 performs the control of the exposure amount changing unit 32 to sequentially change the exposure amount for anterior segment imaging to a plurality of different exposure amounts (exposure amount change control). The imaging controller 71 performs the control of the photographing optical system 3 to acquire time-series images of the anterior segment (imaging control).

Combining the focus position change control, the exposure amount change control, and the imaging control allows the photographing optical system 3 to acquire, for each of the plurality of different focus positions switched by the focus position change control, a plurality of images (first image group) respectively corresponding to the plurality of different exposure amounts switched by the exposure amount change control. As a result, a plurality of first image groups respectively corresponding to the plurality of different focus positions are acquired.

The data processor 8 is configured to perform various types of data processing operations. Data processed by the data processor 8 may be any of data acquired by the ophthalmic apparatus 1 and data input from outside the ophthalmic apparatus 1.

The data processor 8 includes a processor, a main memory, and an auxiliary storage device. The auxiliary storage device stores computer programs such as various data processing programs. These computer programs may be stored in a computer or storage device accessible by the ophthalmic apparatus 1. The functions of the data processor 8 are implemented by cooperation between software such as the data processing programs and hardware such as the processor.

The data processor 8 includes an image processor 81 (see FIG. 2). The image processor 81 is configured to generate a single image (a single anterior segment front image) by performing compositing on the plurality of first image groups respectively corresponding to the plurality of focus positions, which are acquired under the above-described combination control performed by the imaging controller 71. Several examples of the image processor 81 will be described below together with operations of the ophthalmic apparatus 1 that can be performed using the examples of the image processor 81.

The first example of the image processor 81 will be described. The image processor 81 of the present example first applies high dynamic range compositing to each of the plurality of first image groups acquired under the above-described combination control. This process generates a plurality of high dynamic range images respectively corresponding to the plurality of first image groups, in other words, a plurality of high dynamic range images respectively corresponding to the plurality of different focus positions.

Further, the image processor 81 of the present example applies focus compositing to the plurality of high dynamic range images to generate a single anterior segment front image.

In this manner, the image processor 81 of the present example is configured to perform the combination of high dynamic range compositing corresponding to each focus position and focus compositing corresponding to the plurality of different focus positions, thereby generating a single anterior segment front image in which a plurality of regions of the anterior segment are depicted with respective appropriate brightness and in focus.

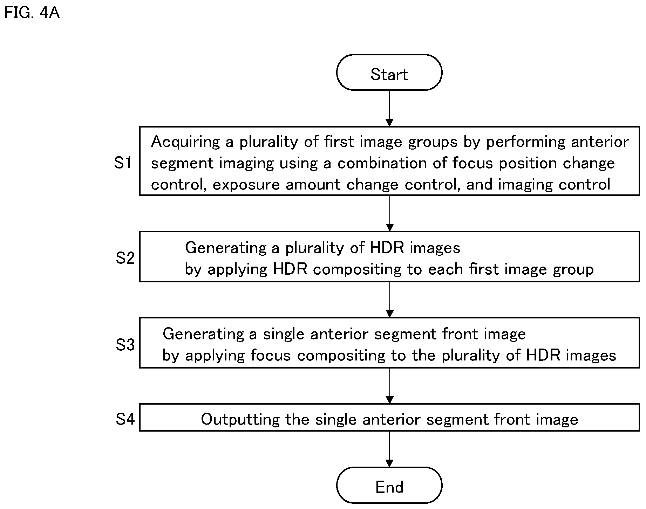

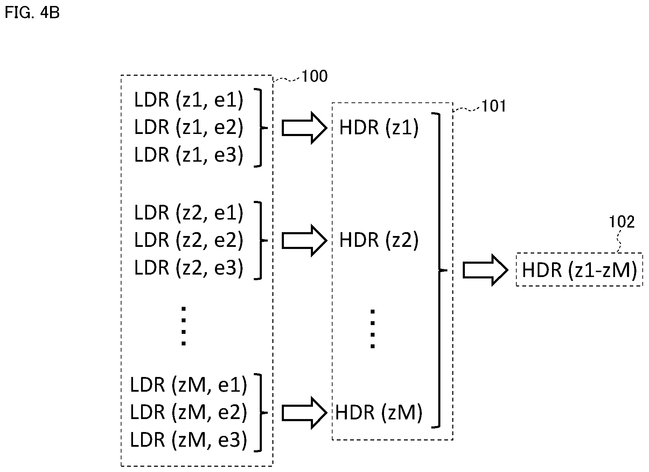

An operation example that can be performed using the image processor 81 of the present example will be described with further reference to FIGs. 4A and 4B. It is assumed that preparatory operations such as alignment and focusing are performed in advance.

As shown in FIG. 4A, initially, the imaging controller 71 performs the combination control including the focus position change control for the focus position changing unit 31, the exposure amount change control for the exposure amount changing unit 32, and the imaging control for the photographing optical system 3. By this combination control, the ophthalmic apparatus 1 acquires a plurality of anterior segment front images (a plurality of first image groups) of the subject's eye E (S1).

The combination control in step S1 may further include illumination control for the illumination optical system 2. The illumination control may include freely selected or designed control relating to projection of illumination light onto the anterior segment of the subject's eye E. In some examples, the illumination control may include any one or more of the following: control for turning on and off the output of the illumination light; control for changing optical properties (e.g., intensity, light amount, wavelength, polarization, etc.) of the illumination light; and control for changing projection conditions (e.g., projection position, projection angle, shape of a projection region, dimensions of a projection region, etc.) of the illumination light.

An image group 100 shown in FIG. 4B is an example of M first image groups acquired in step S1. In FIG. 4B, "LDR" indicates an image having a low dynamic range, and "HDR" indicates an image having a high dynamic range. Here, the high and low dynamic ranges may be relative to each other. In some examples, "LDR" may mean that the image has not been generated using high dynamic range compositing or similar image processing, and "HDR" may mean that the image has been generated using high dynamic range compositing or similar image processing.

In order to acquire the image group 100 including M first image groups in the present example, the following control conditions are applied: the condition for the focus position change control is to sequentially switch the focus position of the photographing optical system 3 to M different focus positions z1, z2, ..., zM (where M is an integer greater than or equal to two); the condition for the exposure amount change control is to sequentially switch the exposure amount in anterior segment imaging to three different exposure amounts e1, e2, and e3; and the condition for the imaging control is to perform imaging once (or more times) for each of 3M pairs (zm, en) (where m = 1 to M, n = 1 to 3) corresponding to all combinations of the M different focus positions z1, z2, ..., zM and the three different exposure amounts e1, e2, and e3.

For example, the imaging controller 71 first controls the focus position changing unit 31 to set the focus position of the photographing optical system 3 to the first focus position z1. The imaging controller 71 also controls the photographing optical system 3 to perform imaging of the anterior segment three times while controlling the exposure amount changing unit 32 to sequentially switch the exposure amount to the three different exposure amounts e1, e2, and e3. As a result, three images LDR(z1, e1), LDR(z1, e2), and LDR(z1, e3) corresponding to the first focus position z1 are obtained. These three images LDR(z1, en) (where n = 1 to 3) constitute a first image group corresponding to the first focus position z1.

Next, the imaging controller 71 controls the focus position changing unit 31 to switch the focus position of the photographing optical system 3 from the first focus position z1 to the second focus position z2. The imaging controller 71 also controls the photographing optical system 3 to perform imaging of the anterior segment three times while controlling the exposure amount changing unit 32 to sequentially switch the exposure amount to the three different exposure amounts e1, e2, and e3. As a result, three images LDR(z2, e1), LDR(z2, e2), and LDR(z2, e3) corresponding to the second focus position z2 are obtained. These three images LDR(z2, en) (where n = 1 to 3) constitute a first image group corresponding to the second focus position z2.

Similarly, the imaging controller 71 controls the focus position changing unit 31 to switch the focus position of the photographing optical system 3 from the m-th focus position zm to the (m+1)-th focus position z(m+1). The imaging controller 71 also controls the photographing optical system 3 to perform imaging of the anterior segment three times while controlling the exposure amount changing unit 32 to sequentially switch the exposure amount to the three different exposure amounts e1, e2, and e3.

By performing the above three imaging operations for each of the M focus positions z1 to zM, three images LDR(zm, e1), LDR(zm, e2), and LDR(zm, e3), respectively corresponding to the three different exposure amounts e1, e2, and e3, are obtained for each focus position zm (where m = 1 to M). That is, as shown in FIG. 4B, the image group 100 including M first image groups respectively corresponding to the M focus positions z1 to zM is obtained.

The control procedure for acquiring the M first image groups (100) is not limited to the present example. In some examples, M images LDR(zm, e1) (where m = 1 to M) corresponding to the first exposure amount e1 may be acquired by performing the imaging operation M times while sequentially switching the focus position to M different focus positions z1 to zM with the first exposure amount e1 applied. In a similar manner, M images LDR(zm, e2) (where m = 1 to M) corresponding to the second exposure amount e2 may be acquired by performing the imaging operation M times while sequentially switching the focus position to the M different focus positions z1 to zM with the second exposure amount e2 applied. Further, M images LDR(zm, e3) (where m = 1 to M) corresponding to the third exposure amount e3 may be acquired by performing the imaging operation M times while sequentially switching the focus position to the M different focus positions z1 to zM with the third exposure amount e3 applied.

After acquisition of the plurality of first image groups (S1) is completed, the image processor 81 applies high dynamic range compositing to each of the plurality of first image groups to generate a plurality of high dynamic range images respectively corresponding to the plurality of different focus positions (S2).

In the example shown in FIG. 4B, the image processor 81 generates a high dynamic range image HDR(zm) by applying high dynamic range compositing to the three images LDR(zm, en) (where n = 1 to 3) corresponding to each focus position zm (where m = 1 to M). As a result, M high dynamic range images HDR(zm) (where m = 1 to M) respectively corresponding to the M different focus positions z1 to zM are obtained (see the reference sign 101 in FIG. 4B).

The processing of step S1 and the processing of step S2 may be performed partially in parallel. In some examples, immediately after the three images LDR(z1, en) (where n = 1 to 3) corresponding to the first focus position z1 are acquired, imaging corresponding to the second focus position z2 may be performed while performing high dynamic range compositing of the three images LDR(z1, en).

After generation of the plurality of high dynamic range images respectively corresponding to the plurality of different focus positions (S2) is completed, the image processor 81 applies focus compositing to the high dynamic range images to generate a single anterior segment front image (S3).

In the example shown in FIG. 4B, the image processor 81 generates a single anterior segment front image HDR(z1-zM) by applying focus compositing to M high dynamic range images HDR(zm) (where m = 1 to M) respectively corresponding to M different focus positions z1 to zM (see the reference sign 102 in FIG. 4B).

Each high dynamic range image HDR(zm) is a dynamic-range-extended image generated from the three images LDR(zm, e1), LDR(zm, e2), and LDR(zm, e3) respectively corresponding to the three different exposure amounts. The depth of field of each high dynamic range image HDR(zm) is limited to a narrow range (that is, to a shallow depth of field) including only the m-th focal plane corresponding to the m-th focus position zm.

In contrast, the anterior segment front image HDR(z1-zM) 102, generated by applying focus compositing to the M high dynamic range images HDR(z1) to HDR(zM) indicated by the reference sign 101, is not only a dynamic-range-extended image but also a depth-of-field-extended image having a large depth of field that includes all of the M different focal planes respectively corresponding to the M different focus positions z1 to zM.

The controller 7 of the present example performs control for outputting the anterior segment front image, generated in step S3, in which both dynamic range and depth of field have been extended (S4).

For example, the controller 7 may be configured to control the communication device 9 to transmit the anterior segment front image to an external apparatus, to control the user interface 10 (display device) to display the anterior segment front image, to store the anterior segment front image in a storage device (not shown in the drawings), or to control a printing device (not shown in the drawings) to print the anterior segment front image. Thus, the operation of the present example is completed (End).

According to the ophthalmic apparatus 1 of the present example, the dynamic range can be extended based on the first image group corresponding to each focus position and to the plurality of different exposure amounts, and the depth of field can be extended based on the plurality of first image groups respectively corresponding to the plurality of different focus positions.

According to the example shown in FIG. 4B, the dynamic range can be extended based on the first image groups LDR(zm, en) (where n = 1 to 3) corresponding to each focus position zm and to the three different exposure amounts en (where n = 1 to 3), and the depth of field can be extended based on the image group 100 including M first image groups respectively corresponding to M different focus positions z1 to zM. The extension of the depth of field is performed based on M dynamic-range-extended images respectively corresponding to the M different focus positions z1 to zM.

Accordingly, the ophthalmic apparatus 1 of the present example is capable of generating a single anterior segment front image in which a plurality of regions of the anterior segment are depicted with respective appropriate brightness and in focus. Therefore, the present example is capable of improving the quality of an image obtained by anterior segment front imaging.

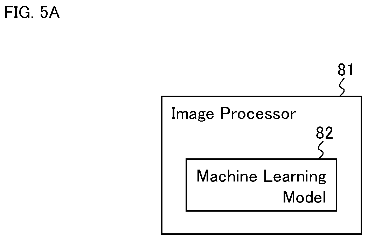

The second example of the image processor 81 will now be described. As shown in FIG. 5A, the image processor 81 of the present example generates a single anterior segment front image from a plurality of first image groups using a machine learning model 82 constructed in advance.

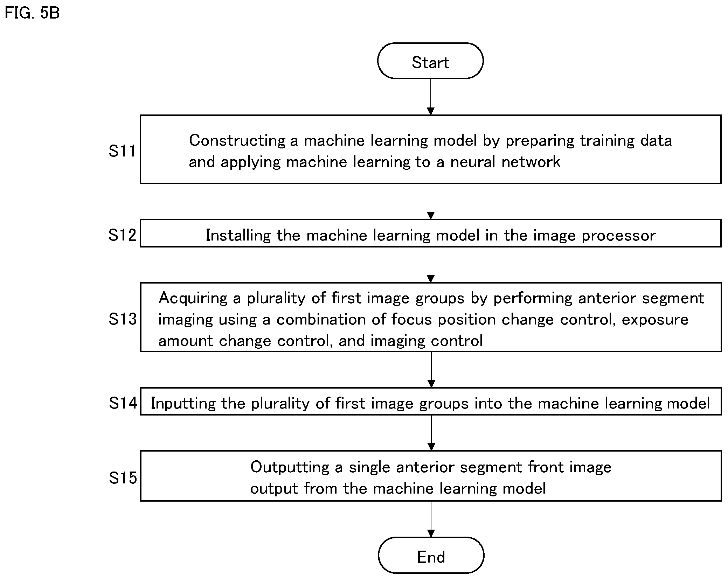

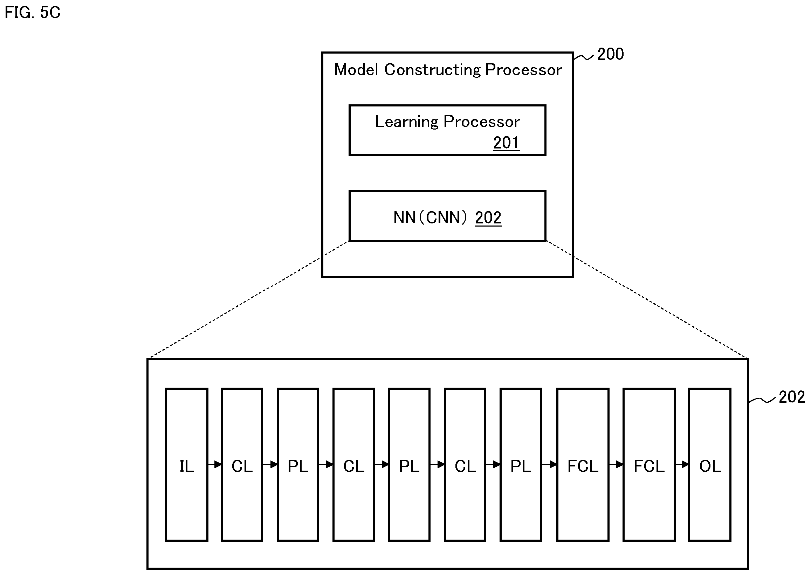

An operation example that can be performed using the image processor 81 of the present example will be described below with further reference to FIGs. 5B and 5C.

As shown in FIG. 5B, training data to be used for constructing the machine learning model 82 is first prepared (S11). The training data may be prepared based on, for example, images of living eyes (and/or images of a model eye; the same applies hereinafter), and includes a plurality of pairs, each pair including a training image group and a training composite image. The training image group includes, for example, a plurality of images acquired by a plurality of times of living eye imaging performed under a plurality of imaging conditions including a plurality of different combinations formed based on a plurality of different focus positions and a plurality of different exposure amounts. That is, the training image group is a set of images each corresponding to a pair of a focus position and an exposure amount. The training composite image is, for example, an image obtained by performing compositing on the training image group. The dynamic range of the training composite image is wider than the dynamic range of each training image, and the depth of field of the training composite image is larger than the depth of field of each training image.

Further, the machine learning model 82 is constructed by applying machine learning based on the training data to a neural network (S11). The neural network may be a freely selected or designed mathematical model that can be used for image processing, and may include, for example, a convolutional neural network.

An example of machine learning for constructing the machine learning model 82 will be described. A device that performs the machine learning (machine learning model construction device) may be disposed in the ophthalmic apparatus 1, or may be disposed in a separate device (e.g., a computer).

A model construction unit 200 shown in FIG. 5C is an example of the machine learning model construction device, and includes a learning processor 201 and a neural network 202.

The neural network 202 of the present example includes a convolutional neural network. The neural network 202 may be constructed using, for example, a known open-source neural network architecture. An example of the structure of the convolutional neural network is shown in FIG. 5C.

An image is input into the input layer of the neural network 202. Subsequent to the input layer, a plurality of pairs of a convolutional layer and a pooling layer is disposed. While three pairs of a convolutional layer and a pooling layer are provided in the neural network 202 shown in FIG. 5C, the number of such pairs may be freely selected or determined.

In the convolutional layer, a convolution operation is performed to detect or extract a feature (e.g., contour) from the input image. This convolution operation is a multiply-accumulate operation (a multiply-add operation, a product-sum operation) on the input image. This multiply-accumulate operation is performed with a filter function (a weight coefficient, a filter kernel) having the same dimension as the input image. In the convolutional layer, the convolution operation is applied to individual parts (individual sections, individual portions, individual regions) of the input image. More specifically, the convolutional layer is configured to calculate a product by multiplying the value of each pixel in a partial image, to which the filter function has been applied, by the value (weight) of the filter function corresponding to this pixel, and then calculate the sum of the products over a plurality of pixels in this partial image. The sum of products obtained in this way is substituted for the corresponding pixel in an image to be output from the convolutional layer. By repetitively performing such multiply-accumulate operation in parallel with moving sites (parts, regions) to which the filter function is applied (that is, in parallel with changing or switching partial images of the input image), a result of the convolution operation for the entire input image is obtained. The convolution operation performed in this way gives a large number of images in which various features have been extracted using a large number of weight coefficients. This means that a large number of filtered images, such as smoothed images and edge images, are obtained. The large number of images generated by the convolutional layer are referred to as feature maps (or activation maps).

The pooling layer executes data compression (e.g., data thinning) of the feature maps generated by the convolutional layer disposed at the immediately preceding position. More specifically, the pooling layer calculates statistical values in predetermined neighboring pixels of a predetermined pixel of interest in an input feature map at each predetermined pixel intervals (stride), and outputs an image having a size smaller than the input feature map. The statistical values applied to the pooling operation may be maximum values (max pooling) or average values (average pooling), for example.

In general, a convolutional neural network extracts many features from an input image by executing processing using a plurality of pairs of a convolutional layer and a pooling layer.

A fully connected layer is disposed subsequent to the most downstream pair of a convolutional layer and a pooling layer. While two fully connected layers are provided in the structural example shown in FIG. 5C, the number of fully connected layers may be freely selected or determined. The fully connected layer executes processing such as image classification, image segmentation, or regression using the features compressed by the combination of convolution and pooling. An output layer is disposed subsequent to the most downstream fully connected layer. The output layer gives an output result.

Some aspects may employ a convolutional neural network including no fully connected layer. For example, some aspects may employ a fully convolutional network (FCN). Some aspects may include a support vector machine, a recurrent neural network (RNN), or any other models. Machine learning applied to the neural network 202 may include transfer learning. Further, the model constructing processor 200 (the learning processor 201) may be configured in such a manner that fine tuning can be applied to the neural network 202.

The learning processor 201 applies machine learning with training data to the neural network 202 (S11). In a case in which the neural network 202 includes a convolutional neural network, parameters tuned by the learning processor 201 include, for example, filter coefficients of one or more convolutional layers, and connection weights and offsets of one or more fully connected layers.

As described above, the training data may include a set (dataset) of pairs of a training image group and a training composite image, in which the training image group consists of a plurality of images acquired by imaging the anterior segment of a living eye a plurality of times under a plurality of imaging conditions including a plurality of combinations of a plurality of focus positions and a plurality of exposure amounts, and the training composite image is generated from the training image group. Each pair included in the training image group is an image of the same type as an image input into the image processor 81 (the machine learning model 82). Therefore, compared with a case in which machine learning is performed using training data consisting only of images of a different type, it is considered that the quality (e.g., accuracy, precision) of the output of the image processor 81 can be improved.

Note that the types of images included in the training data are not limited to images of the same type as images input into the image processor 81. In some examples, the training data may include any one or more of the following types of images: an image acquired using a freely selected or determined ophthalmic modality (e.g., slit lamp microscope, fundus camera, OCT apparatus, surgical microscope); an image acquired using a diagnostic imaging modality of a clinical department other than ophthalmology (e.g., ultrasonic diagnostic apparatus, X-ray diagnostic apparatus, X-ray computed tomography (CT) apparatus, magnetic resonance imaging (MRI) apparatus); an image generated by processing an actual image; and a pseudo image generated by a computer. Further, the number of images included in the training data may be increased by using any technique such as data augmentation.

The machine learning method employed for constructing the machine learning model 82 may be freely selected or designed. In some examples, the machine learning method may be any one method selected from a group including supervised learning, semi-supervised learning, unsupervised learning, and reinforcement learning, or may be a combination, at least in part, of any two or more methods selected from the group. In some aspects, supervised learning (or semi-supervised learning) is conducted using training data in which a label as a final output is assigned to each input image. In some examples, supervised learning may be performed using training data including a training image group as input images and training composite images as labels.

The machine learning model 82 constructed in this manner functions to receive input of a plurality of anterior segment front images (first image groups) respectively corresponding to a plurality of different combinations of a plurality of different focus positions and a plurality of different exposure amounts, and to output a composite image generated from the plurality of anterior segment front images, with both dynamic range and depth of field extended.

The methods and techniques used for constructing the machine learning model 82 are not limited to the examples described above. In some examples, any methods and techniques such as the following options may be employed for constructing a machine learning model: support vector machine, Bayesian classifier, boosting, k-means clustering, kernel density estimation, principal component analysis, independent component analysis, self-organizing map, random forest, and generative adversarial network (GAN).

The machine learning model 82 constructed as described above is installed in the image processor 81 (S12). Therefore, the image processor 81 becomes capable of using the machine learning model 82. The machine learning model 82 may be included in the image processor 81, or may be included in a computer accessible by the image processor 81. The steps described up to this point are preparation steps performed before anterior segment imaging of the subject's eye E.

Next, anterior segment imaging of the subject's eye E is performed (S13). Specifically, similarly to step S1 in FIG. 4A, the imaging controller 71 performs the combination control including the focus position change control for the focus position changing unit 31, the exposure amount change control for the exposure amount changing unit 32, and the imaging control for the photographing optical system 3. The combination control allows the ophthalmic apparatus 1 to acquire a plurality of anterior segment front images (a plurality of first image groups) of the subject's eye E.

The plurality of first image groups acquired in step S13 are sent to the image processor 81. The image processor 81 inputs the plurality of first image groups into the machine learning model 82 (S14). The machine learning model 82 generates a single anterior segment front image based on the plurality of first image groups input. Compared with the first image groups input, the anterior segment front image has both an extended dynamic range and an extended depth of field.

The controller 7 performs control for outputting the anterior segment front image generated in step S14 (S15), similarly to step S4 in FIG. 4A. Thus, the operation of the present example is completed (End).

As described above, the ophthalmic apparatus 1 of the present example is configured to input a plurality of first image groups respectively corresponding to a plurality of different combinations of a plurality of different focus positions and a plurality of different exposure amounts into the machine learning model, thereby generating an anterior segment front image with both extended dynamic range and extended depth of field. The ophthalmic apparatus 1 of the present example configured in this manner is capable of generating an anterior segment front image in which a plurality of regions of the anterior segment are depicted with respective appropriate brightness and in focus. Accordingly, the quality of an image obtained by anterior segment front imaging can be improved.

The third example of the image processor 81 will be described. Instead of compositing all images included in a plurality of first image groups acquired by anterior segment imaging, the present example is configured to composite only a plurality of images selected from a plurality of first image groups to generate an anterior segment front image with both dynamic range and depth of field extended.

The first image groups may include images acquired during blinking or images contaminated by unwanted light. Performing image compositing including such images may cause deterioration in the quality of a resulting image. One objective of the present example is to address this issue.

The ophthalmic apparatus 1 according to the present embodiment example acquires a plurality of first image groups by sequentially imaging a plurality of in-focus regions respectively corresponding to a plurality of different focus positions (depth regions corresponding to depth of fields). It should be noted that the number of types of anterior segment tissues included in each focus region differs from one another. For example, an in-focus region in imaging performed with a focus position placed at or near the corneal apex includes only the cornea, whereas an in-focus region in imaging performed with a focus position placed at or near the anterior surface of the crystalline lens includes the crystalline lens and the iris. Further, in an event that two or more types of anterior segment tissues are included in an in-focus region, there may be cases in which it is desirable or sufficient to clearly depict only one of the anterior segment tissues. On the other hand, since anterior segment tissues have inherent optical properties (e.g., reflectance, scattering characteristics, etc.), the brightness of images obtained by imaging at a constant exposure amount varies depending on the types of anterior segment tissues. Accordingly, for example, there may be cases in which imaging once with one exposure amount is sufficient at a certain focus position, whereas imaging two or more times with two or more exposure amounts is desirable at another focus position. Therefore, there may be cases in which high dynamic range compositing is not necessary at a certain focus position, whereas high dynamic range compositing is desirable at another focus position. More generally, the number of images required for a certain focus position and the number of images applied to another focus position may differ from each other. Alternatively, the number of exposure amounts (the number of imaging operations) applied to a certain focus position and the number of exposure amounts (the number of imaging operations) applied to another focus position may differ from each other. One objective of the present example is to achieve improvements in this regard.

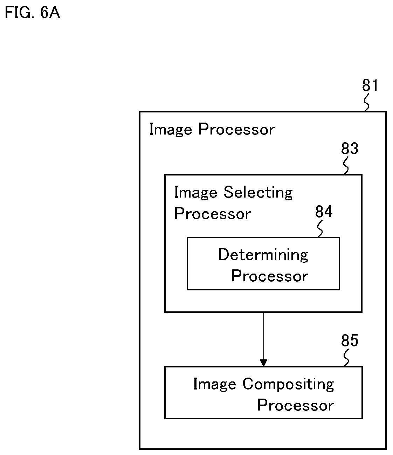

As shown in FIG. 6A, the image processor 81 of the present example includes an image selecting processor 83 and an image compositing processor 85. The image selecting processor 83 includes a determining processor 84.

The image selecting processor 83 selects one or more images from each of a plurality of first image groups acquired by the photographing optical system 3 under the combination of the focus position change control, the exposure amount change control, and the imaging control performed by the imaging controller 71. In other words, the image selecting processor 83 selects one or more images from the first image group corresponding to each focus position applied in the focus position change control. A set of images selected from the plurality of first image groups by the image selecting processor 83 is referred to as a selected image group. The image compositing processor 85 composites the selected image group to generate a single anterior segment front image having both extended dynamic range and extended depth of field.

Contents of the processing performed by the image selecting processor 83 to extract the selected image group from the plurality of first image groups may be freely selected or designed. In the present example, the image selecting processor 83 selects one image from a first image group corresponding to a focus position located on a focal plane that intersects only one type of anterior segment tissue, and selects two or more images from a first image group corresponding to a focus position located on a focal plane that intersects two or more different types of anterior segment tissues. In other words, the image selecting processor 83 of the present example changes the number of images selected from each first image group based on the number of types of anterior segment tissues intersecting a focal plane. The number of types of anterior segment tissues intersecting the focal plane may be defined to be the number of types of anterior segment tissues intersecting the corresponding focal plane itself, or the number of types of anterior segment tissues included within a depth-of-field range (in-focus region) that includes the corresponding focal plane. The image selecting processor 83 of the present example is configured to perform such image selection processing using the determining processor 84; however, the image selection processing is not limited to this configuration.

The determining processor 84 is configured to determine, for each of the plurality of different focus positions applied during anterior segment imaging, whether or not the focal plane including the focus position intersects a plurality of different anterior segment tissues by analyzing an image corresponding to the focus position. This image analysis may include a freely selected or designed image analysis method that can be used to identify an anterior segment image. In some examples, the image analysis may include image segmentation (image segmentation using a machine learning model, and/or image segmentation using an algorithm that does not use machine learning).

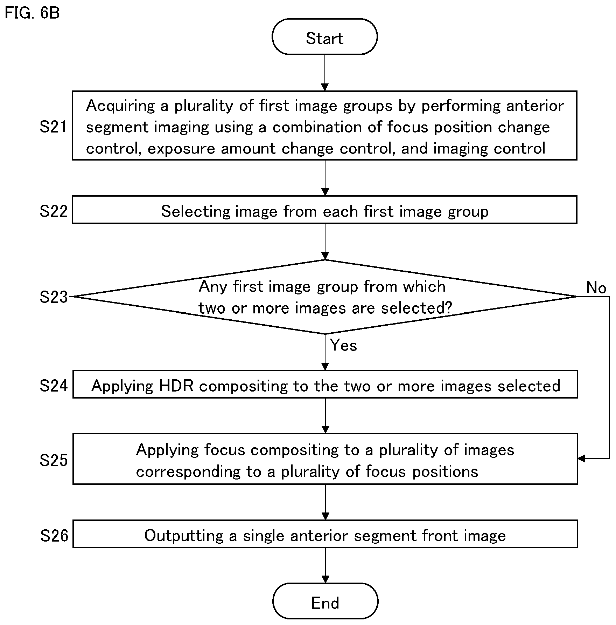

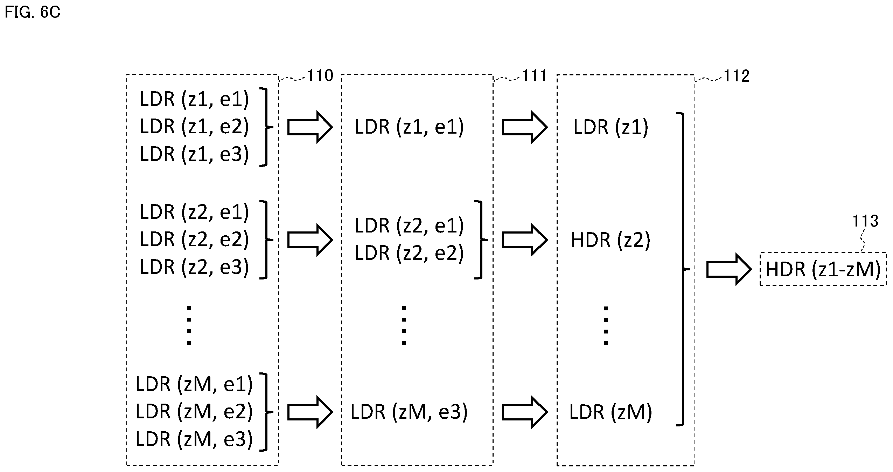

An operation example that can be performed using the image processor 81 of the present example will be described with further reference to FIGs. 6B and 6C.

As shown in FIG. 6B, the present operation example begins with anterior segment imaging of the subject's eye E (S21). Specifically, similarly to step S1 of FIG. 4A, the imaging controller 71 performs the combination control including the focus position change control for the focus position changing unit 31, the exposure amount change control for the exposure amount changing unit 32, and the imaging control for the photographing optical system 3. By this combination control, the ophthalmic apparatus 1 acquires a plurality of anterior segment front images (a plurality of first image groups) of the subject's eye E. The plurality of first image groups acquired in step S21 are transmitted to the image processor 81.

The image selecting processor 83 selects one or more images from each of the plurality of first image groups acquired for the plurality of different focus positions in step S21 (S22). As a result, a selected image group is obtained.

In step S22, the determining processor 84 determines, for each focus position applied in the anterior segment imaging of step S21, whether or not the focal plane including the focus position intersects two or more different anterior segment tissues, by analyzing an image corresponding to the focus position. The determining processor 84 makes this determination for each focus position by analyzing one image or two or more images included in the corresponding first image group. In some examples, the determining processor 84 may analyze each image included in the first image group to determine the quality (suitability, validity, etc.) of the image, and then make the above determination for one image or two or more images that have been determined to suitable quality. Here, the quality of the image may be determined based on, for example, whether or not the image has been acquired during blinking, or whether or not the image includes an image of unwanted light.

The image selecting processor 83 of the present example may be configured to select one image from a first image group corresponding to a focus position included in a focal plane that is determined by the determining processor 84 to intersect only one type of anterior segment tissue, and select two or more images from a first image group corresponding to a focus position included in a focal plane that is determined by the determining processor 84 to intersect two or more different anterior segment tissues. In the latter case, the number of images selected may be freely determined and may be, in some examples, a number determined in advance. Alternatively, the image selecting processor 83 may be configured to determine the number of images to be selected from the first image group based on the number of anterior segment tissues intersecting the focal plane. In either case, the image selecting processor 83 may be configured to select only images of suitable quality, excluding images obtained during blinking, images containing an image of unwanted light, and the like.

The selected image group obtained in step S22 is sent to the image compositing processor 85. If there is a first image group from which two or more images have been selected (S23: Yes), the image compositing processor 85 applies high dynamic range compositing to the two or more images selected from that first image group (S24). The high dynamic range compositing may be performed using a machine learning model, and/or using an algorithm that does not use machine learning. If one image has been selected from each of all first image groups (S23: No), step S24 is not performed.

Through the above processing, a plurality of anterior segment front images respectively corresponding to the plurality of different focus positions applied in the anterior segment imaging of step S21 are obtained. In other words, the above processing generates one anterior segment image for each focus position. If high dynamic range compositing in step S24 has been performed for one or more first image groups, one or more of the plurality of anterior segment front images obtained are high dynamic range images. Considering the structure of the anterior segment, a portion of the cornea and a portion of the sclera exist at substantially the same depth position, and a portion of the crystalline lens and a portion of the iris exist at substantially the same depth position. Accordingly, in practice, an anterior segment front image corresponding to a focus position is a high dynamic range image.

The image compositing processor 85 applies focus compositing to the plurality of anterior segment front images obtained to generate a single anterior segment front image (S25). The focus compositing may be performed using a machine learning model, and/or may be performed using an algorithm that does not use machine learning. The anterior segment front image generated in step S25 is an image having extended depth of field, and also having extended dynamic range (in practice).

The image compositing processor 85 of the present example may be configured to perform, using a machine learning model, image processing (image generation) that is a combination (integration) of the high dynamic range compositing in step S24 and the focus compositing in step S25.

The controller 7 performs control for outputting the anterior segment front image generated in step S25 (S26), similarly to step S4 in FIG. 4A. Thus, the operation of the present example is completed (End).

One specific example of the operation shown in FIG. 6B will be described with further reference to FIG. 6C. In the present example, the anterior segment imaging of step S21 yields an image group (a plurality of first image groups) indicated by the reference sign 110 as shown in FIG. 6C. The image group 110 is a set of images similar to the image group 100 of FIG. 4B, and includes M first image groups respectively corresponding to M focus positions z1 to zM. Each first image group consists of three images LDR(zm, e1), LDR(zm, e2), and LDR(zm, e3) respectively corresponding to three different exposure amounts e1, e2, and e3.

In the example shown in FIG. 6C, a selected image group 111 is obtained from the image group 110 by the image selecting processor 83 in step S22. The selected image group 111 is obtained by the following image selection processing: one image LDR(z1, e1) is selected from the three images LDR(z1, e1), LDR(z1, e2), and LDR(z1, e3) included in the first image group corresponding to the first focus position z1; two images LDR(z2, e1) and LDR(z2, e2) are selected from the three images LDR(z2, e1), LDR(z2, e2), and LDR(z2, e3) included in the first image group corresponding to the second focus position z2; and one image LDR(zM, e3) is selected from the three images LDR(zM, e1), LDR(zM, e2), and LDR(zM, e3) included in the first image group corresponding to the M-th focus position zM.

Here, it is assumed that one image is selected from each of M-3 first image groups respectively corresponding to the third through the (M-1)-th focus positions. In this case, the number of images included in the selected image group 111 is M+1.

In the example shown in FIG. 6C, the image compositing processor 85 applies high dynamic range compositing to the two images LDR(z2, e1) and LDR(z2, e2) both corresponding to the second focus position z2, thereby generating a high dynamic range image HDR(z2). This processing is carried out in step S24.

As a result, the image group 112 is obtained. The image group 112 includes the high dynamic range image HDR(z2) corresponding to the second focus position z2, and images LDR(zm) corresponding to each of the other focus positions zm (where m = 1 to M, m ≠ 2). Here, the images LDR(zm) in the image group 112 are respectively the same as the images LDR(zm, en) in the selected image group 111 (where m = 1 to M, m ≠ 2; and n = 1, 2, or 3).

In the example shown in FIG. 6C, the image compositing processor 85 applies focus compositing to the M images included in the image group 112, thereby generating the anterior segment front image HDR(z1-zM) 113. This processing is carried out in step S25.

The anterior segment front image HDR(z1-zM) 113 is an image in which the dynamic range in the depth region corresponding to the second focus position z2 is extended, and the depth of field throughout the depth regions respectively corresponding to the plurality of different focus positions z1 to zM is extended.

Therefore, in the anterior segment front image HDR(z1-zM) 113, a plurality of types of anterior segment tissues located in the depth region corresponding to the second focus position z2 are depicted with respective appropriate brightness and in focus. In addition, one anterior segment tissue located in the depth regions respectively corresponding to the other focus positions zm (m ≠ 2) is also depicted with appropriate brightness and in focus.

In this way, the ophthalmic apparatus 1 of the present example is capable of generating a single anterior segment front image in which a plurality of regions of the anterior segment are depicted with respective appropriate brightness and in focus. Accordingly, the quality of an image obtained by anterior segment front imaging can be improved.

In the present example, the ophthalmic apparatus 1 is configured to acquire a plurality of images (a plurality of first image groups) respectively corresponding to a plurality of different combinations of a plurality of different focus positions and a plurality of different exposure amounts, extract a selected image group from the plurality of first image groups, and perform image compositing. With this configuration, the ophthalmic apparatus 1 of the present example provides a characteristic advantageous effect in that a composite image can be generated with defective images excluded.

In contrast, when a plurality of different focus positions to be applied in anterior segment imaging are predetermined, the number of imaging operations at each focus position may be determined in advance based on the relationship between these focus positions and the structure of the anterior segment. Here, the number of imaging operations at each focus position is, in other words, the number and values of exposure amounts to be applied. Accordingly, for example, in a case corresponding to the specific example shown in FIG. 6C, imaging may be performed twice (with exposure amounts e1 and e2) at the second focus position z2, and imaging may be performed once at each of the other focus positions zm (with a default exposure amount). This results in obtaining an image group equivalent to the selected image group 111. According to the present example, it becomes possible to achieve advantageous effects such as reduction of imaging time, reduction of the burden imposed on the subject, and improvement of examination throughput.

Another aspect of the ophthalmic apparatus 1 will be described. The configuration of the ophthalmic apparatus 1 of the present aspect may be the same as the configuration shown in FIGs. 1 to 3B.

In the above aspect, the ophthalmic apparatus 1 acquires an image group (a plurality of first image groups) corresponding to all combinations of a plurality of different focus positions and a plurality of different exposure amounts, by performing the combination of the focus position change control, the exposure amount change control, and the imaging control. Accordingly, in the above aspect, the anterior segment imaging is performed (at least) a number of times equal to the product of the number of the plurality of different focus positions and the number of the plurality of different exposure amounts.

In contrast, in the present aspect, the ophthalmic apparatus 1 performs the combination of the focus position change control, the exposure amount change control, and the imaging control, to acquire an image group (a second image group) corresponding to a plurality of pairs in which each pair includes a focus position and an exposure amount that are associated with each other based on a one-to-one correspondence between a plurality of different focus positions and a plurality of different exposure amounts. In other words, the ophthalmic apparatus 1 acquires the second image group including a plurality of images respectively corresponding to a plurality of pairs including a pair consisting of a focus position and an exposure amount that are associated with each other based on a bijection defined between a set of a plurality of different focus positions and a set of a plurality of different exposure amounts, by performing a combination of the focus position change control, the exposure amount change control, and the imaging control. Since the bijection is defined between the set of focus positions and the set of exposure amounts, the numbers of elements of both sets are equal to one another. Therefore, the number of imaging operations performed in anterior segment imaging of the present aspect is equal to the numbers of elements of the two sets. Assuming that the number of focus positions (the number of exposure amounts) applied in anterior segment imaging is the same between the above aspect and the present aspect, the number of imaging operations in the present aspect is less than that in the above aspect.

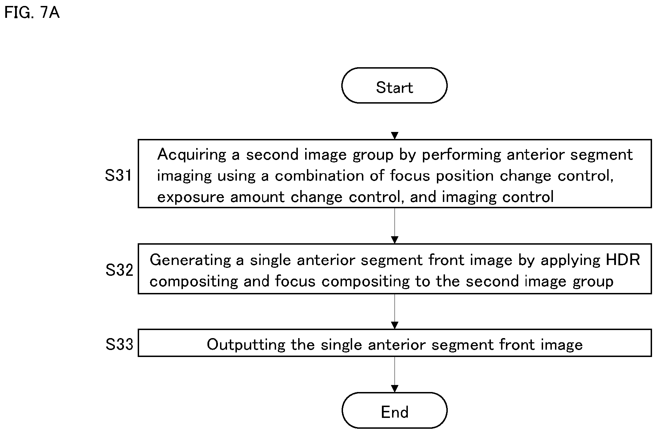

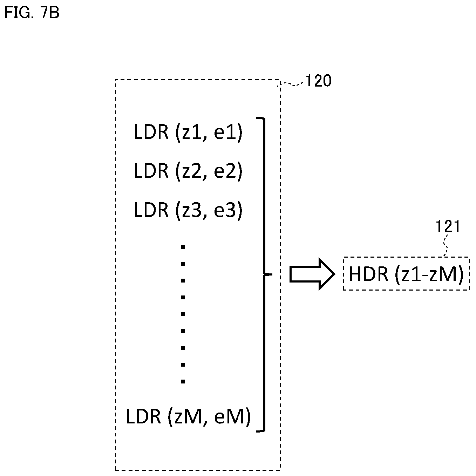

An operation example that can be performed by the ophthalmic apparatus 1 of the present aspect will be described with further reference to FIGs. 7A and 7B.

As shown in FIG. 7A, the present operation example begins with anterior segment imaging of the subject's eye E (S31). The imaging controller 71 of the present aspect performs combination control including the focus position change control for the focus position changing unit 31, the exposure amount change control for the exposure amount changing unit 32, and the imaging control for the photographing optical system 3, in a similar manner to step S1 of FIG. 4A. However, unlike step S1 of FIG. 4A, the combination control of step S31 is performed in such a manner as to acquire a second image group corresponding to a plurality of pairs including a pair of a focus position and an exposure amount that are associated with each other based on a one-to-one correspondence between a plurality of different focus positions and a plurality of different exposure amounts.

For example, as shown in FIG. 7B, the ophthalmic apparatus 1 of the present aspect performs anterior segment imaging a plurality of times while sequentially applying a plurality of pairs (zm, em) of a focus position and an exposure amount, thereby acquiring the second image group 120 consisting of the plurality of images LDR(z1, e1), LDR(z2, e2), ..., LDR(zM, eM) respectively corresponding to the plurality of pairs (zm, em). The second image group acquired in step S31 is sent to the image processor 81.

The image processor 81 generates a single anterior segment front image by performing compositing on the second image group acquired in step S31 (S32). This image compositing processing includes both processing of extending dynamic range and processing of extending depth of field. In the example shown in FIG. 7B, the anterior segment front image HDR(z1-zM) 121 is generated by applying image compositing processing to the second image group 120.

In some examples, the image processor 81 may be configured to perform, using a machine learning model, image processing (image generation) that integrates high dynamic range compositing and focus compositing. In some other examples, the image processor 81 may be configured to generate a dynamic-range-extended image (high dynamic range image) by applying high dynamic range compositing to the second image group, generate a depth-of-field-extended image by applying focus compositing to the second image group, and generate a single anterior segment front image by performing compositing on the dynamic-range-extended image and the depth-of-field-extended image. The image compositing processing that can be performed in step S32 is not limited to these examples.

The anterior segment front image generated in step S32 with both dynamic range and depth of field extended is sent to the controller 7. Similarly to step S4 in FIG. 4A, the controller 7 performs control for outputting the anterior segment front image (S33). Thus, the operation of the present aspect is completed (End).

As described above, the ophthalmic apparatus 1 of the present example is capable of generating a single anterior segment front image in which a plurality of regions of the anterior segment are depicted with respective appropriate brightness and in focus. Accordingly, it becomes possible to improve the quality of an image obtained by front imaging of the anterior segment. Another advantage is that the number of anterior segment imaging operations can be reduced.

In the present aspect described above, the imaging controller 71 is configured to perform the combination of the focus position change control, the exposure amount change control, and the imaging control. The focus position change control controls the focus position changing unit 31 to sequentially change the focus position of the photographing optical system 3 to a plurality of different focus positions. The exposure amount change control controls the exposure amount changing unit 32 to sequentially change the exposure amount for imaging of the anterior segment to "a plurality of different exposure amounts". The imaging control controls the photographing optical system 3 to acquire time-series images. By performing such combination control, the imaging controller 71 causes the photographing optical system 3 to acquire the second image group corresponding to the plurality of pairs determined based on a one-to-one correspondence between the plurality of different focus positions and "the plurality of different exposure amounts" (that is, corresponding to the plurality of pairs of a focus position and an exposure amount). Therefore, the plurality of images in the second image group respectively correspond to different exposure amounts.

In contrast, in some examples, two (or three or more) images included in the second image group may correspond to the same exposure amount. In this case, the imaging controller 71 is configured to perform the combination of the following control processes: the focus position change control that controls the focus position changing unit 31 to sequentially change the focus position of the photographing optical system 3 to a plurality of different focus positions; the exposure amount change control that controls the exposure amount changing unit 32 to sequentially change the exposure amount for imaging of the anterior segment to "a plurality of exposure amounts (two or more exposure amounts may have the same value)"; and the imaging control that controls the photographing optical system 3 to acquire time-series images. By performing such combination control, the imaging controller 71 causes the photographing optical system 3 to acquire the second image group corresponding to the plurality of pairs determined based on a one-to-one correspondence between the plurality of different focus positions and "the plurality of exposure amounts" (i.e., the plurality of pairs of a focus position and an exposure amount). Typically, the number of different exposure amounts is less than or equal to the number of different focus positions. The present example is capable of improving image quality while reducing the number of imaging operations.

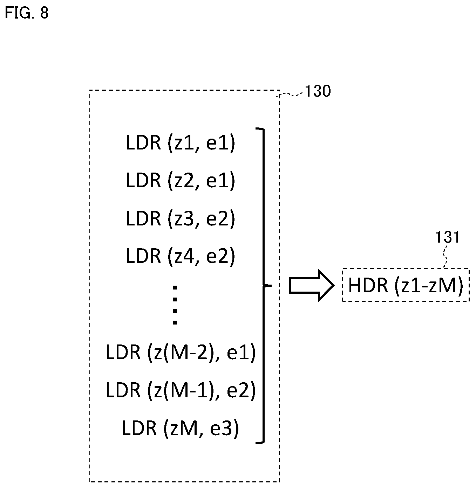

In anterior segment imaging for acquiring the second image group 130 shown in FIG. 8, the exposure amount corresponding to the focus position z1, the exposure amount corresponding to the focus position z2, and the exposure amount corresponding to the focus position z(M-2) have the same value e1. Further, the exposure amount corresponding to the focus position z3, the exposure amount corresponding to the focus position z4, and the exposure amount corresponding to the focus position z(M-1) have the same value e2. In the present example, a single anterior segment front image in which both dynamic range and depth of field are extended is generated by performing compositing on the second image group 130.

In the present aspect, each pair of a focus position and an exposure amount may be a combination of the following: a focus position corresponding to one anterior segment tissue among a plurality of different anterior segment tissues; and an exposure amount determined in advance based on the scattering intensity of that anterior segment tissue. By determining a plurality of such pairs according to the present example, default imaging conditions corresponding to the scattering characteristics of the plurality of different anterior segment tissues (e.g., cornea, crystalline lens, iris) can be prepared. Accordingly, anterior segment imaging can be facilitated and simplified.

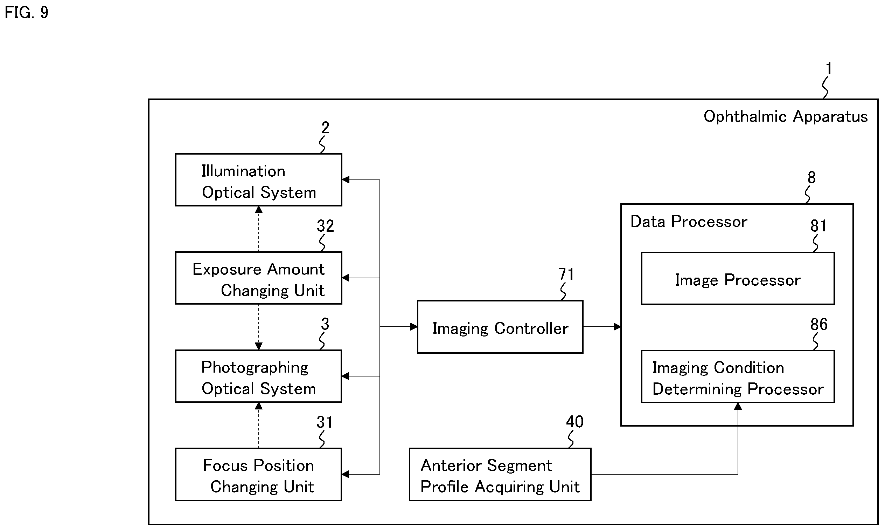

Instead of or in addition to preparing default imaging conditions, the ophthalmic apparatus 1 of the present aspect may have a function of determining imaging conditions (one or more pairs of a focus position and an exposure amount). A configuration example of the ophthalmic apparatus 1 having the imaging condition determining function is shown in FIG. 9. The configuration of FIG. 9 is obtained by adding an anterior segment profile acquiring unit 40 and an imaging condition determining processor 86 to the configuration shown in FIG. 2.

The anterior segment profile acquiring unit 40 acquires a depth profile of the anterior segment of the subject's eye E. The depth profile may be a freely designed type of information indicating a distribution of anterior segment data in the eye axis direction (Z direction). For example, the depth profile may be any one or more of the following: an anterior segment cross-sectional image obtained by a slit lamp microscope, anterior segment data obtained by OCT scanning (e.g., A-scan data, an A-scan image, a B-scan image, a three-dimensional image, etc.), anterior segment data obtained by ultrasound scanning (e.g., A-scan data, an A-scan image, a B-scan image, a three-dimensional image, etc.), and anterior segment data obtained by axial length measurement.

The anterior segment profile acquiring unit 40 includes, for example, a depth profile generating processor configured to generate a depth profile and/or a depth profile receiving unit configured to receive a depth profile from an external source. The depth profile generating processor may be, for example, an element of the data processor 8. The functions of the depth profile receiving unit may be implemented by using, for example, the communication device 9 configured to receive a depth profile from an external device, a reading device configured to read a depth profile from a recording medium, or an image scanner configured to convert a depth profile recorded on a printed medium into digital data.

The imaging condition determining processor 86 determines a plurality of pairs of a focus position and an exposure amount to be applied in anterior segment imaging of the subject's eye E, based on the depth profile of the anterior segment of the subject's eye E acquired by the anterior segment profile acquiring unit 40.

The depth profile acquired by the anterior segment profile acquiring unit 40 includes information indicating the positions of various anterior segment tissues in the subject's eye E (tissue position information), and/or images of various anterior segment tissues (tissue images). In some examples, A-scan data of the anterior segment includes a peak indicating the position of the cornea (e.g., anterior corneal surface, posterior corneal surface), a peak indicating the position of the crystalline lens (e.g., anterior capsule, posterior capsule), or like features. An image of the anterior segment includes an image of the cornea, an image of the anterior chamber, an image of the iris, an image of the crystalline lens, or like images.

When the depth profile includes tissue position information, as in the case of A-scan data, the imaging condition determining processor 86 identifies depth positions of a plurality of different anterior segment tissues of the subject's eye E by using, for example, processing of detecting tissue position information from the depth profile. This processing may include signal processing such as peak detection. Further, the imaging condition determining processor 86 determines a plurality of pairs of a focus position and an exposure amount based on the plurality of depth positions respectively identified for the plurality of different anterior segment tissues of the subject's eye E and on scattering intensity information of these anterior segment tissues. The scattering intensity information may be, for example, standard values of scattering intensity of the anterior segment tissues, or measured values of scattering intensity of the anterior segment tissues obtained through measurement using OCT or other methods.

When the depth profile includes tissue images, as in the case of images obtained by a slit lamp microscope or an OCT apparatus, the imaging condition determining processor 86 identifies depth positions of a plurality of different anterior segment tissues of the subject's eye E by using, for example, processing of detecting tissue images (positions of the tissue images) from the depth profile. This processing may include image processing such as segmentation. Further, the imaging condition determining processor 86 determines a plurality of pairs of a focus position and an exposure amount based on the plurality of depth positions respectively identified for the plurality of different anterior segment tissues of the subject's eye E and on scattering intensity information of these anterior segment tissues.

According to the present example, a plurality of pairs of a focus position and an exposure amount can be determined as imaging conditions based on anterior segment data (the depth profile) of the subject's eye E, and a second image group can be acquired using these imaging conditions. Therefore, imaging can be performed under conditions appropriate for each individual subject's eye.

As described above, the plurality of pairs of a focus position and an exposure amount may include only a plurality of pairs determined based on a one-to-one correspondence between a plurality of different focus positions and a plurality of different exposure amounts, or may further include pairs different from those pairs. In the former case, one image is acquired for each focus position. In the latter case, for example, two or more images may be acquired for a certain focus position. The example illustrated in FIG. 8 described above (a case where the number of different focus positions applied in anterior segment imaging is greater than the number of different exposure amounts applied) corresponds to another example of the latter case.

In the present aspect, an example in which two or more images are acquired for a certain focus position will be described. The imaging controller 71 of the present example is configured to cause the photographing optical system to acquire, for each of one or more focus positions among the plurality of different focus positions, two or more images respectively corresponding to two or more different exposure amounts. The second image group of the present example includes two or more images corresponding to each of the above one or more focus positions among the plurality of different focus positions, and includes one image corresponding to each of another one or more focus positions.

The imaging controller 71 of the present example may be configured to cause the photographing optical system to acquire two or more images corresponding to two or more different exposure amounts for a focus position located on a focal plane that intersects a plurality of different anterior segment tissues. This configuration may be the same as the similar example described above.

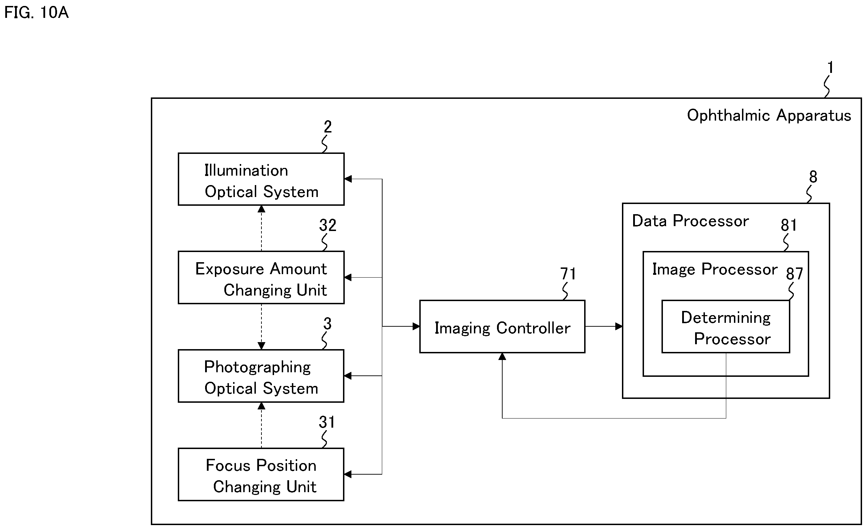

For this purpose, the image processor 81 of the present example may include a determining processor 87 (see FIG. 10A). The determining processor 87 is configured to determine, for each of the plurality of different focus positions, whether or not the focal plane including the focus position intersects a plurality of different anterior segment tissues. The configuration and operation of the determining processor 87 of the present example may be the same as the configuration and operation of the determining processor 84 of FIG. 6A. The determining processor 87 of the present example may be configured to analyze an image corresponding to each focus position in order to perform the above determination. Here, the image corresponding to each focus position may include, for example, an anterior segment image of the subject's eye E, an anterior segment image of another eye, or an anterior segment image of a standard eye model.

The imaging controller 71 of the present example performs control (combination control of the focus position change control, the exposure amount change control, and the imaging control) for causing the photographing optical system 3 to acquire the second image group based on a determination result obtained by the determining processor 87. As a result, for a focus position corresponding to a focal plane determined to intersect a plurality of different anterior segment tissues, two or more imaging operations respectively corresponding to two or more different exposure amounts are performed, and two or more images are acquired. On the other hand, for a focus position corresponding to a focal plane determined to intersect only one anterior segment tissue, one or more imaging operations corresponding to one exposure amount are performed, and one image is acquired. A plurality of images acquired through such a series of imaging operations form the second image group of the present example.

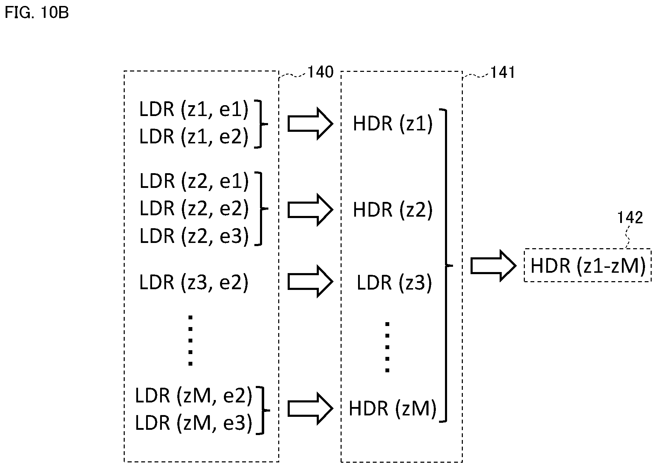

A specific example of the present example will be described. The second image group 140 of FIG. 10B includes the following images: for the focus position z1, two images LDR(z1, e1) and LDR(z1, e2) respectively corresponding to two different exposure amounts e1 and e2 are included; for the focus position z2, three images LDR(z2, e1), LDR(z2, e2), and LDR(z2, e3) respectively corresponding to three different exposure amounts e1, e2, and e3 are included; for the focus position z3, one image LDR(z3, e2) corresponding to one exposure amount e2 is included; and for the focus position zM, two images LDR(zM, e2) and LDR(zM, e3) respectively corresponding to two different exposure amounts e2 and e3 are included. Although not shown in the drawings, for each of the focus positions other than the focus positions z1, z2, z3 and zM, one or more images respectively corresponding to one or more exposure amounts are included.

The image processor 81 of the present example generates a high dynamic range image by applying high dynamic range compositing to two or more corresponding images for a focus position corresponding to a focal plane determined to intersect a plurality of different anterior segment tissues. The high dynamic range image generated is used for focus compositing. On the other hand, one image acquired for a focus position corresponding to a focal plane determined to intersect only one anterior segment tissue is used directly for focus compositing. The image group 141 of FIG. 10B includes a plurality of images acquired through such processing.

Further, the image processor 81 of the present example generates a single anterior segment front image by applying focus compositing to the image group including one or more high dynamic range images generated in the manner described above. The image HDR(z1-zM) 142 of FIG. 10B corresponds to the single anterior segment front image generated in this way.

The present example is capable of generating an anterior segment front image in which both dynamic range and depth of field are extended, and automatically determining a focus position (depth region) to which high dynamic range compositing is to be applied in anterior segment imaging of each individual subject's eye.

One modification example, as in a similar example of the aspect described above, may be configured to perform the following processes: a process of setting a focus position to which high dynamic range compositing is to be applied (a default focus position) in advance; a process of acquiring two or more images respectively corresponding to two or more different exposure amounts in the imaging at the focus position set in advance; a process of generating a high dynamic range image from the two or more images acquired; and a process of generating a single anterior segment front image based on an image group including the high dynamic range image generated.

In the present aspect, the method of processing of generating a single anterior segment front image from the second image group (image compositing) may be freely selected or determined. For example, the image compositing may be performed using a machine learning model. The image processor 81 of the present example includes a machine learning model (see FIG. 5A). The machine learning model of the present example is trained by machine learning to receive input of a plurality of images having mutually different focus positions and mutually different exposure amounts, and to output a composite image of the plurality of images input. When the second image group of the present example is input into the machine learning model, the machine learning model outputs a single anterior segment front image that is a composite image of the second image group.

The machine learning model of the present example may have a freely selected or designed configuration. For example, the machine learning model may include a convolutional neural network constructed by supervised learning using predetermined training data. The training data may include, for example, a plurality of pairs, each pair including a training image group having mutually different focus positions and mutually different exposure amounts and a training composite image that is a composite image of this training image group.

In another example, the technique or technology described in the following document may be used: Xin Huang et al., "Inverting the Imaging Process by Learning an Implicit Camera Model," https://doi.org/10.48550/arXiv.2304.12748, submitted on 25 Apr 2023.

The image compositing may be performed without using machine learning. For example, the image processor 81 of the present example may be configured to generate a single anterior segment front image by applying image processing that includes both focus compositing and high dynamic range compositing to the second image group. The image processing of the present example may be performed using, for example, the technique or technology described in the following document: Qinchun Qian and Bahadir K. Gunturk, "Extending depth of field and dynamic range from differently focused and exposed images," Multidimensional Systems and Signal Processing, 27, 493-509 (2016), https://doi.org/10.1007/s11045-015-0315-x.

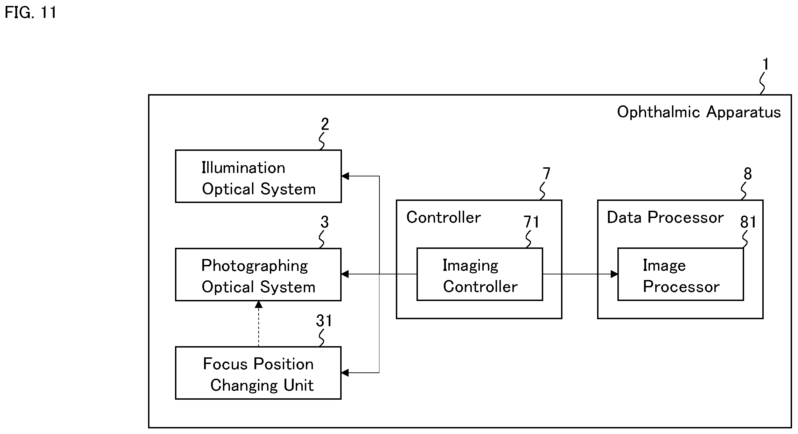

Yet another aspect of the ophthalmic apparatus 1 will be described. The configuration of the ophthalmic apparatus 1 of the present aspect may be the same as that shown in FIGs. 1 to 3B; however, the ophthalmic apparatus 1 of the present aspect may not include the exposure amount changing unit 32 as shown in FIG. 11.

In the above aspects, the ophthalmic apparatus 1 acquires an image group by performing the focus position change control, the exposure amount change control, and the imaging control in combination. In contrast, the ophthalmic apparatus 1 of the present aspect acquires an image group (a third image group) by performing the focus position change control and the imaging control in combination without performing the exposure amount change control.

In the focus position change control of the present aspect, the imaging controller 71 controls the focus position changing unit 31 to sequentially change the focus position of the photographing optical system 3 to a plurality of different focus positions. Furthermore, in the imaging control of the present aspect, the imaging controller 71 controls the photographing optical system 3 to acquire time-series images at a constant exposure amount. The anterior segment imaging of the present aspect differs from that of the above aspects in that imaging is performed at a constant exposure amount.

By combining such focus position change control and imaging control, the imaging controller 71 causes the photographing optical system 3 to acquire the third image group corresponding to the plurality of different focus positions. The third image group includes a plurality of images respectively corresponding to the plurality of different focus positions. The third image group includes only one image for each focus position. When the plurality of different focus positions are z1, z2, ..., zM (M in number), the third image group includes M images. The image processor 81 generates a single anterior segment front image by performing compositing on the third image group.

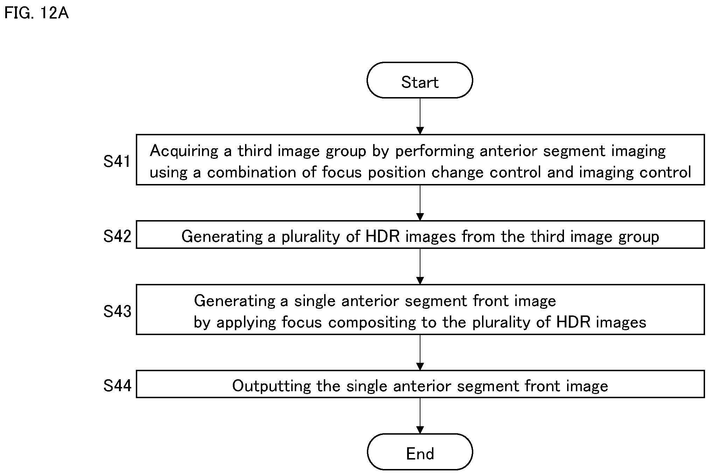

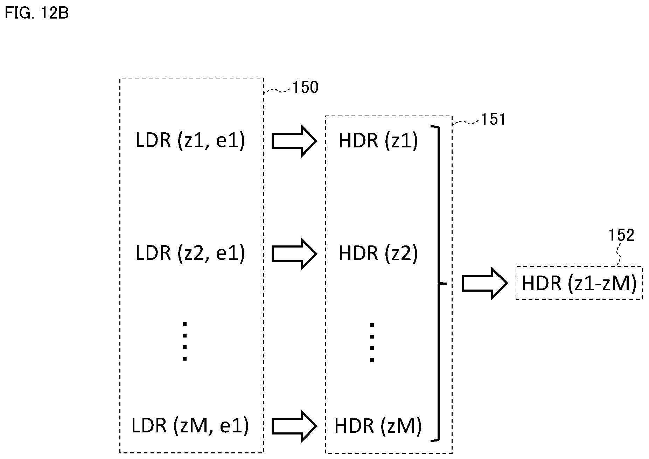

An operation example that can be performed by the ophthalmic apparatus 1 of the present aspect will be described with further reference to FIGs. 12A and 12B.

As shown in FIG. 12A, the operation example begins with anterior segment imaging of the subject's eye E (S41). Unlike step S1 of FIG. 4A, the imaging controller 71 of the present aspect performs combination control of the focus position change control for the focus position changing unit 31 and the imaging control for the photographing optical system 3. A constant exposure amount (the same exposure amount) is applied in the plurality of imaging operations performed in the anterior segment imaging. The constant exposure amount may be a predetermined default exposure amount or an exposure amount determined based on various conditions relating to imaging such as a condition or state of the subject's eye E or a main observation target region. Through step S41, the ophthalmic apparatus 1 of the present aspect acquires the third image group corresponding to the plurality of different focus positions.

For example, as shown in FIG. 12B, the ophthalmic apparatus 1 of the present aspect performs M imaging operations at M focus positions z1, z2, ..., zM with a constant exposure amount e1, thereby acquiring the third image group 150 including M images LDR(z1, e1), LDR(z2, e1), ..., LDR(zM, e1) respectively corresponding to M focus positions z1, z2, ..., zM. The third image group acquired in step S41 is sent to the image processor 81.

The image processor 81 generates a plurality of high dynamic range images respectively corresponding to a plurality of different focus positions from the third image group acquired in step S41 (S42). In the example shown in FIG. 12B, the high dynamic range image group 151 including M high dynamic range images HDR(z1), HDR(z2), ..., HDR(zM) is generated from the M images LDR(z1, e1), LDR(z2, e1), ..., LDR(zM, e1). That is, for each of the plurality of focus positions zm (where m = 1 to M), a high dynamic range image HDR(zm) is generated from the image LDR(zm, e1) corresponding to the focus position zm.

The high dynamic range image generation in step S42 differs from typical high dynamic range compositing that composites a plurality of images acquired by imaging with a plurality of different exposure amounts. Instead, the high dynamic range image generation in step S42 is processing of extending the dynamic range of a single image, in other words, processing of generating a pseudo high dynamic range image.

Various methods and techniques have been proposed for this type of processing, many of which utilize machine learning techniques. The present aspect may use the technique described in the following document, for example: Lin Wang and Kuk-Jin Yoon, "Deep Learning for HDR Imaging: State-of-the-Art and Future Trends," JOURNAL OF LATEX CLASS FILES, VOL. 14, NO. 8, AUGUST 2015, https://doi.org/10.48550/arXiv.2110.10394. In addition, as described in this document, the present aspect may use a technique based on the number or region of input exposures, a technique based on the number of learning tasks, a technique based on novel sensor data, a technique based on novel learning strategies, a technique based on applications, or like techniques.

The image processor 81 of the present aspect includes a machine learning model. This machine learning model is trained by machine learning to receive input of one image having a first dynamic range and to output an image having a second dynamic range greater than the first dynamic range. By inputting each image in the third image group into this machine learning model, a plurality of high dynamic range images respectively corresponding to the plurality of different focus positions are acquired.

Next, the image processor 81 generates a single anterior segment front image by applying focus compositing to the plurality of high dynamic range images generated in step S42 (S43). This anterior segment front image is an image in which both dynamic range and depth of field have been extended. In the example shown in FIG. 12B, the anterior segment front image HDR(z1-zM) 152 is generated by applying focus compositing to the high dynamic range image group 151.

The anterior segment front image generated in step S43 is sent to the controller 7. The controller 7 performs control for outputting this anterior segment front image (S44) in the same manner as in step S4 in FIG. 4A. Thus, the operation of the present example is completed (End).

As described above, the ophthalmic apparatus 1 of the present aspect is capable of generating a single anterior segment front image in which a plurality of regions of the anterior segment are depicted with respective appropriate brightness and in focus. This improves the quality of an image obtained by front imaging of the anterior segment. Another advantage is that the number of imaging operations for anterior segment imaging may be reduced. Further, since exposure amount change control is not performed in anterior segment imaging, there is also an advantage that control can be facilitated and simplified.

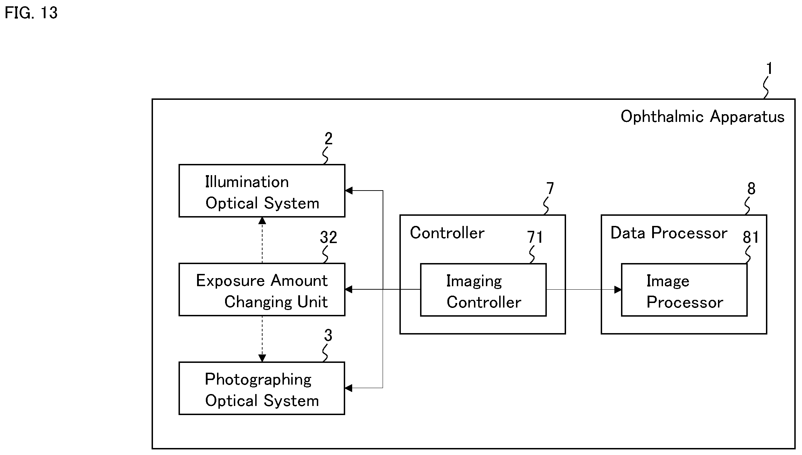

Still another aspect of the ophthalmic apparatus 1 will be described. The configuration of the ophthalmic apparatus 1 of the present aspect may be the same as that shown in FIGs. 1 to 3B; however, the ophthalmic apparatus 1 of the present aspect may not include the focus position changing unit 31 as shown in FIG. 13.

In the aspects shown in FIGs. 2, 9, and 10A, the ophthalmic apparatus 1 acquires an image group by performing the focus position change control, the exposure amount change control, and the imaging control in combination. Also, in the aspect shown in FIG. 11, the ophthalmic apparatus 1 acquires an image group by performing the focus position change control and the imaging control in combination. In contrast, the ophthalmic apparatus 1 of the present aspect shown in FIG. 13 acquires an image group (a fourth image group) by performing the exposure amount change control and the imaging control in combination without performing the focus position change control.

In the exposure amount change control of the present aspect, the imaging controller 71 controls the exposure amount changing unit 32 to sequentially change the exposure amount for imaging of the anterior segment to a plurality of different exposure amounts. In the imaging control of the present aspect, the imaging controller 71 controls the photographing optical system 3 to acquire time-series images at a constant focus position. The anterior segment imaging of the present aspect differs from that of the above aspects in that imaging is performed at a constant focus position.

By combining such exposure amount change control and imaging control, the imaging controller 71 causes the photographing optical system 3 to acquire the fourth image group corresponding to the plurality of different exposure amounts. The fourth image group includes a plurality of images respectively corresponding to the plurality of different exposure amounts. The fourth image group includes only one image for each exposure amount. When the plurality of different exposure amounts are e1, e2, ..., eN (N in number), the fourth image group includes N images. The image processor 81 generates a single anterior segment front image by performing compositing on the fourth image group.

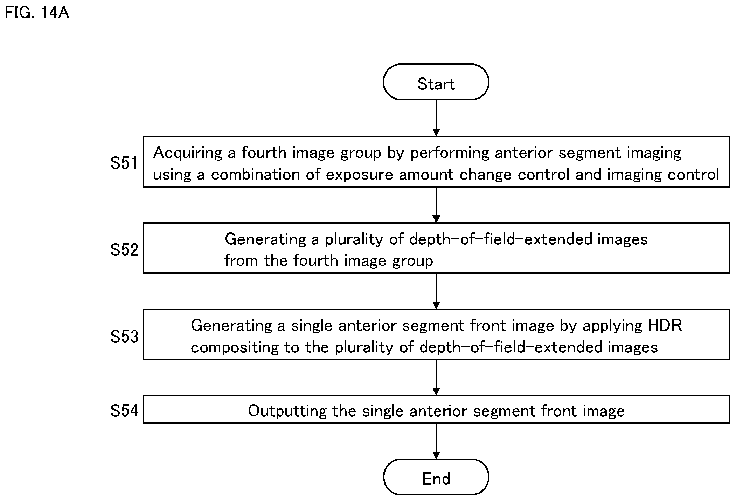

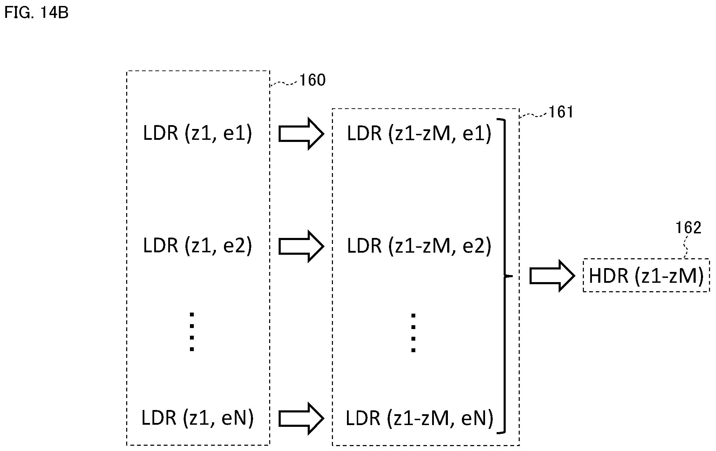

An operation example that can be performed by the ophthalmic apparatus 1 of the present aspect will be described with further reference to FIGs. 14A and 14B.

As shown in FIG. 14A, the operation example begins with anterior segment imaging of the subject's eye E (S51). Unlike step S1 of FIG. 4A, the imaging controller 71 of the present aspect performs combination control of the exposure amount change control for the exposure amount changing unit 32 and the imaging control for the photographing optical system 3. A constant focus position (the same focus position) is applied in the plurality of imaging operations performed in the anterior segment imaging. The focus position may be a predetermined default focus position or a focus position determined based on various conditions relating to imaging such as a condition or state of the subject's eye E or a main observation target region. Through step S51, the ophthalmic apparatus 1 of the present aspect acquires the fourth image group corresponding to the plurality of different exposure amounts.

For example, as shown in FIG. 14B, the ophthalmic apparatus 1 of the present aspect performs N imaging operations at N exposure amounts e1, e2, ..., eN with a constant focus position z1, thereby acquiring the fourth image group 160 including N images LDR(z1, e1), LDR(z1, e2), ..., LDR(z1, eN) respectively corresponding to N exposure amounts e1, e2, ..., eN. The fourth image group acquired in step S51 is sent to the image processor 81.

The image processor 81 generates a plurality of depth-of-field-extended images respectively corresponding to the plurality of different exposure amounts from the fourth image group acquired in step S51 (S52). In the example of FIG. 14B, a depth-of-field-extended image group 161 including N depth-of-field-extended images LDR(z1-zM, e1), LDR(z1-zM, e2), ..., LDR(z1-zM, eN) is generated from the N images LDR(z1, e1), LDR(z1, e2), ..., LDR(z1, eN). That is, for each of the plurality of exposure amounts en (where n = 1 to N), a depth-of-field-extended image LDR(z1-zM, en) is generated from the image LDR(z1, en) corresponding to the exposure amount en. Here, the image LDR(z1, en) corresponding to the exposure amount en has a depth of field corresponding to the focus position z1. The depth-of-field-extended image LDR(z1-zM, en) generated from the image LDR(z1, en) has a large depth of field corresponding to M different focus positions z1 to zM.

The depth-of-field extension in step S52 differs from typical depth-of-field extension (focus compositing) that composites a plurality of images acquired by imaging at a plurality of different focus positions. Instead, the depth-of-field extension in step S52 is processing of extending the depth of field of a single image, in other words, processing of generating a pseudo depth-of-field-extended image.

Various methods and techniques have been proposed for this type of processing, many of which utilize machine learning techniques. In the present aspect, for example, the technique for extending the depth of field using a phase mask and deep learning described in the following document may be adopted: Lingbo Jin et al., "Deep learning extended depth-of-field microscope for fast and slide-free histology," The Proceedings of the National Academy of Sciences (PNAS), December 14, 2020, 117 (52) 33051-33060, https://doi.org/10.1073/pnas.2013571117.

The image processor 81 of the present aspect includes a machine learning model. This machine learning model is trained by machine learning to receive input of one image having a first depth of field and to output an image having a second depth of field larger than the first depth of field. By inputting each image in the fourth image group into this machine learning model, a plurality of depth-of-field-extended images respectively corresponding to the plurality of different exposure amounts are acquired.

Next, the image processor 81 generates a single anterior segment front image by applying high dynamic range compositing to the plurality of depth-of-field-extended images generated in step S52 (S53). This anterior segment front image is an image in which both dynamic range and depth of field have been extended. In the example shown in FIG. 14B, the anterior segment front image HDR(z1-zM) 162 is generated by applying high dynamic range compositing to the depth-of-field-extended image group 161.

The anterior segment front image generated in step S53 is sent to the controller 7. The controller 7 performs control for outputting this anterior segment front image (S54) in the same manner as in step S4 in FIG. 4A. Thus, the operation of the present example is completed (End).

As described above, the ophthalmic apparatus 1 of the present aspect is capable of generating a single anterior segment front image in which a plurality of regions of the anterior segment are depicted with respective appropriate brightness and in focus. This improves the quality of an image obtained by front imaging of the anterior segment. Another advantage is that the number of imaging operations for anterior segment imaging can be reduced. Further, since focus position change control is not performed in anterior segment imaging, there is also an advantage that control can be facilitated and simplified.

Several non-limiting aspects of the ophthalmic apparatus according to the embodiment examples are described above. Any two or more of these aspects may be combined at least in part.

### < Other Aspects >

Embodiment examples according to the present disclosure are not limited to an ophthalmic apparatus. Embodiment examples other than the ophthalmic apparatus include a method of controlling an ophthalmic apparatus, a method of processing an image acquired by an ophthalmic apparatus, a method of acquiring and processing an image of an eye, a program, and a recording medium. As in the embodiment examples of the ophthalmic apparatus, these embodiment examples are also capable of improving image quality in anterior segment imaging.

Some embodiment examples provide a method for controlling an ophthalmic apparatus. The first aspect of the method for controlling the ophthalmic apparatus will be described. The ophthalmic apparatus of the present aspect may be used for imaging an anterior segment of a subject's eye. The ophthalmic apparatus includes an illumination optical system, a photographing optical system, a focus position changing unit, an exposure amount changing unit, and a processor. The illumination optical system is configured to project illumination light onto the anterior segment of the subject's eye. The photographing optical system is configured to perform imaging of the anterior segment, from a frontal position relative to the subject's eye, onto which the illumination light is projected. The focus position changing unit is configured to change a focus position of the photographing optical system. The exposure amount changing unit is configured to change an exposure amount for imaging of the anterior segment. The method of the present aspect includes an imaging step and an image processing step. The imaging step is carried out by the processor performing combination control of focus position change control, exposure amount change control, and imaging control. In the focus position change control, the processor controls the focus position changing unit to sequentially change the focus position of the photographing optical system to a plurality of different focus positions. In the exposure amount change control, the processor controls the exposure amount changing unit to sequentially change the exposure amount for imaging of the anterior segment to a plurality of different exposure amounts. In the imaging control, the processor controls the photographing optical system to acquire time-series images. The imaging step of the present aspect causes the processor to perform the combination control to cause the photographing optical system to acquire, for each of the plurality of different focus positions, a first image group corresponding to the plurality of different exposure amounts. In the image processing step, the processor generates a single image by performing compositing on a plurality of first image groups acquired in the imaging step and respectively corresponding to the plurality of different focus positions.

The second aspect of the method for controlling the ophthalmic apparatus will be described. The ophthalmic apparatus of the present aspect may be used for imaging an anterior segment of a subject's eye. As in the first aspect, the ophthalmic apparatus includes an illumination optical system, a photographing optical system, a focus position changing unit, an exposure amount changing unit, and a processor. The method of the present aspect includes an imaging step and an image processing step. The imaging step is carried out by the processor performing combination control of focus position change control, exposure amount change control, and imaging control. These controls are the same as those in the first aspect, respectively. The imaging step of the present aspect causes the processor to perform the combination control, thereby causing the photographing optical system to acquire a second image group corresponding to a plurality of pairs, each pair including a focus position and an exposure amount and the plurality of pairs including a pair based on a one-to-one correspondence between the plurality of different focus positions and the plurality of different exposure amounts. In the image processing step, the processor generates a single image by performing compositing on the second image group acquired in the imaging step.

The third aspect of the method for controlling the ophthalmic apparatus will now be described. The ophthalmic apparatus of the present aspect may be used for imaging an anterior segment of a subject's eye. As in the first aspect, the ophthalmic apparatus includes an illumination optical system, a photographing optical system, a focus position changing unit, and a processor. However, the ophthalmic apparatus of the present aspect does not include an exposure amount changing unit. The method of the present aspect includes an imaging step and an image processing step. The imaging step is carried out by the processor performing combination control of focus position change control and imaging control. These controls are the same as those in the first aspect, respectively. Note that the imaging control in the present aspect is performed at a constant exposure amount (the same exposure amount). The imaging step of the present aspect causes the processor to perform the combination control described above, thereby causing the photographing optical system to acquire the third image group corresponding to the plurality of different focus positions. The third image group includes only one image for each of the plurality of different focus positions. In the image processing step, the processor generates a single image by performing compositing on the third image group acquired in the imaging step.

The fourth aspect of the method for controlling the ophthalmic apparatus will now be described. The ophthalmic apparatus of the present aspect may be used for imaging an anterior segment of a subject's eye. As in the first aspect, the ophthalmic apparatus includes an illumination optical system, a photographing optical system, an exposure amount changing unit, and a processor. However, the ophthalmic apparatus of the present aspect does not include a focus position changing unit. The method of the present aspect includes an imaging step and an image processing step. The imaging step is carried out by the processor performing combination control of exposure amount change control and imaging control. These controls are the same as those in the first aspect, respectively. Note that the imaging control in the present aspect is performed at a constant focus position (the same focus position). The imaging step of the present aspect causes the processor to perform the combination control described above, thereby causing the photographing optical system to acquire the fourth image group corresponding to the plurality of different exposure amounts. The fourth image group includes only one image for each of the plurality of different exposure amounts. In the image processing step, the processor generates a single image by performing compositing on the fourth image group acquired in the imaging step.

Any matters or items described in the present disclosure may be combined with the methods according to the embodiment examples.

Some embodiment examples provide a program. The program according to an embodiment example causes a computer including a processor and a memory to perform the method according to any one of the first to fourth aspects described above. Any matters or items described in the present disclosure may be combined with the program according to the embodiment example.

Some embodiment examples provide a computer-readable non-transitory recording medium. The recording medium according to an embodiment example stores a program that causes a computer to perform the method according to any one of the first to fourth aspects described above. Any matters or items described in the present disclosure may be combined with the recording medium according to the embodiment example.

The computer-readable non-transitory recording medium that can be used as the recording medium according to the embodiment example may be a recording medium in any form. Examples of the recording medium include a magnetic disk, an optical disk, a magnetooptical disk, a semiconductor memory, and any other types of recording media.

The embodiment examples and aspects described in the present disclosure are merely illustrative examples. Any modifications (e.g., omissions, substitutions, additions, or the like) within the scope of the present invention can be applied to the embodiment examples and aspects of the present disclosure.

### [REFERENCE SIGNS LIST]

- 1: Ophthalmic apparatus
- 2: Illumination optical system
- 3: Photographing optical system
- 31: Focus position changing unit
- 32: Exposure amount changing unit
- 32A: Exposure time changing unit
- 32B: Illumination intensity changing unit
- 40: Anterior segment profile acquiring unit
- 7: Controller
- 71: Imaging controller
- 8: Data processor
- 81: Image processor
- 82: Machine learning model
- 83: Image selecting processor
- 84: Determining processor
- 85: Image compositing processor
- 86: Imaging condition determining processor
- 87: Determining processor

## Claims

1. An ophthalmic apparatus comprising:
an illumination optical system configured to project illumination light onto an anterior segment of a subject's eye;
a photographing optical system configured to perform imaging of the anterior segment, from a front side, onto which the illumination light is projected;
a focus position changing unit configured to change a focus position of the photographing optical system;
an exposure amount changing unit configured to change an exposure amount for imaging of the anterior segment;
an imaging controller configured to perform a combination of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire, for each of the plurality of different focus positions, a first image group corresponding to the plurality of different exposure amounts; and
an image processor configured to generate a single image by performing compositing on a plurality of first image groups respectively corresponding to the plurality of different focus positions.

2. The ophthalmic apparatus of claim 1, wherein the image processor generates a plurality of high dynamic range images respectively corresponding to the plurality of different focus positions by applying high dynamic range compositing to each of the plurality of first image groups, and generates the single image by applying focus compositing to the plurality of high dynamic range images.

3. The ophthalmic apparatus of claim 1, wherein the image processor includes
an image selecting processor configured to select one or more images from each of the plurality of first image groups, and
an image compositing processor configured to generate the single image by performing compositing on a selected image group selected from the plurality of first image groups by the image selecting processor.

4. The ophthalmic apparatus of claim 3, wherein based on two or more images being selected from each of one or more first image groups among the plurality of first image groups by the image selecting processor, the image compositing processor generates, for each of the one or more first image groups, one or more high dynamic range images respectively corresponding to the one or more first image groups by applying high dynamic range compositing to the two or more images selected from the first image group to generate a high dynamic range image, and to generate the single image by applying focus compositing to an image group including the one or more high dynamic range images.

5. The ophthalmic apparatus of claim 4, wherein the image selecting processor is configured to select two or more images from a first image group corresponding to a focus position located on a focal plane that intersects a plurality of different anterior segment tissues.

6. The ophthalmic apparatus of claim 5, wherein the image selecting processor includes a determining processor configured to determine, for each of the plurality of different focus positions, whether a focal plane including the focus position intersects a plurality of different anterior segment tissues by analyzing an image corresponding to the focus position.

7. An ophthalmic apparatus comprising:
an illumination optical system configured to project illumination light onto an anterior segment of a subject's eye;
a photographing optical system configured to perform imaging of the anterior segment, from a front side, onto which the illumination light is projected;
a focus position changing unit configured to change a focus position of the photographing optical system;
an exposure amount changing unit configured to change an exposure amount for imaging of the anterior segment;
an imaging controller configured to perform a combination of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire a second image group corresponding to a plurality of pairs, each pair including a focus position and an exposure amount and the plurality of pairs including a pair based on a one-to-one correspondence between the plurality of different focus positions and the plurality of different exposure amounts; and
an image processor configured to generate a single image by performing compositing on the second image group.

8. The ophthalmic apparatus of claim 7, wherein each of the plurality of pairs includes a focus position corresponding to one anterior segment tissue among a plurality of different anterior segment tissues and an exposure amount determined in advance based on a scattering intensity of the anterior segment tissue.

9. The ophthalmic apparatus of claim 7, further comprising
an anterior segment profile acquiring unit configured to acquire a depth profile of the anterior segment; and
an imaging condition determining processor configured to determine the plurality of pairs based on the depth profile.

10. The ophthalmic apparatus of claim 9, wherein the imaging condition determining processor is configured to identify depth positions of a plurality of different anterior segment tissues based on the depth profile, and to determine the plurality of pairs based on the depth positions of the plurality of different anterior segment tissues and scattering intensity information of the plurality of different anterior segment tissues.

11. The ophthalmic apparatus of any one of claims 7 to 10, wherein
the imaging controller is configured to cause the photographing optical system to acquire, for each of one or more focus positions among the plurality of different focus positions, two or more images respectively corresponding to two or more different exposure amounts,
the second image group includes, for each of one or more focus positions, the two or more images corresponding to that focus position, and
the image processor is configured to generate a high dynamic range image for each of the one or more focus positions by applying high dynamic range compositing to two or more images corresponding to the focus position and to generate the single image based on an image group including one or more high dynamic range images respectively corresponding to the one or more focus positions.

12. The ophthalmic apparatus of claim 11, wherein the imaging controller is configured to cause the photographing optical system to acquire the two or more images for a focus position located on a focal plane that intersects a plurality of different anterior segment tissues.

13. The ophthalmic apparatus of claim 12, wherein
the image processor includes a determining processor configured to determine, for each of the plurality of different focus positions, whether a focal plane including the focus position intersects a plurality of different anterior segment tissues, and
the imaging controller is configured to perform control for causing the photographing optical system to acquire the second image group based on a determination result obtained by the determining processor.

14. The ophthalmic apparatus of any one of claims 7 to 10, wherein the image processor includes a machine learning model trained by machine learning to receive input of a plurality of images having mutually different focus positions and mutually different exposure amounts, and to output a composite image of the plurality of images, and
the machine learning model is configured to generate the single image from the second image group.

15. The ophthalmic apparatus of claim 14, wherein the machine learning model includes a convolutional neural network trained by supervised learning using training data including a plurality of pairs, each pair including a training image group having mutually different focus positions and mutually different exposure amounts and a training composite image that is a composite image of the training image group.

16. The ophthalmic apparatus of any one of claims 7 to 10, wherein the image processor is configured to generate the single image by applying image processing including both focus compositing and high dynamic range compositing to the second image group.

17. An ophthalmic apparatus comprising:
an illumination optical system configured to project illumination light onto an anterior segment of a subject's eye;
a photographing optical system configured to perform imaging of the anterior segment, from a front side, onto which the illumination light is projected;
a focus position changing unit configured to change a focus position of the photographing optical system;
an imaging controller configured to perform a combination of focus position change control that controls the focus position changing unit to sequentially change the focus position of the photographing optical system to a plurality of different focus positions, and imaging control that controls the photographing optical system to acquire time-series images at a constant exposure amount, thereby causing the photographing optical system to acquire a third image group corresponding to the plurality of different focus positions; and
an image processor configured to generate a single image by performing compositing on the third image group,
wherein the third image group includes only one image for each of the plurality of different focus positions.

18. The ophthalmic apparatus of claim 17, wherein the image processor is configured to generate a plurality of high dynamic range images respectively corresponding to the plurality of different focus positions by generating a high dynamic range image from each of a plurality of images included in the third image group, and to generate the single image by applying focus compositing to the plurality of high dynamic range images.

19. The ophthalmic apparatus of claim 18, wherein the image processor includes a machine learning model trained by machine learning to receive input of one image having a first dynamic range and to output an image having a second dynamic range greater than the first dynamic range, and
the machine learning model is configured to generate the plurality of high dynamic range images from the third image group.

20. An ophthalmic apparatus comprising:
an illumination optical system configured to project illumination light onto an anterior segment of a subject's eye;
a photographing optical system configured to perform imaging of the anterior segment, from a front side, onto which the illumination light is projected;
an exposure amount changing unit configured to change an exposure amount for imaging of the anterior segment;
an imaging controller configured to perform a combination of exposure amount change control that controls the exposure amount changing unit to sequentially change the exposure amount for imaging of the anterior segment to a plurality of different exposure amounts, and imaging control that controls the photographing optical system to acquire time-series images at a constant focus position, thereby causing the photographing optical system to acquire a fourth image group corresponding to the plurality of different exposure amounts; and
an image processor configured to generate a single image by performing compositing on the fourth image group,
wherein the fourth image group includes only one image for each of the plurality of different exposure amounts.

21. The ophthalmic apparatus of claim 20, wherein the image processor is configured to generate a plurality of depth-of-field-extended images respectively corresponding to the plurality of different exposure amounts by generating a depth-of-field-extended image from each of a plurality of images included in the fourth image group, and to generate the single image by applying high dynamic range compositing to the plurality of depth-of-field-extended images.

22. The ophthalmic apparatus of claim 21, wherein the image processor includes a machine learning model trained by machine learning to receive input of one image having a first depth of field and to output an image having a second depth of field larger than the first depth of field, and
the machine learning model is configured to generate the plurality of depth-of-field-extended images from the fourth image group.

23. The ophthalmic apparatus of any one of claims 1, 7, and 20, wherein
the exposure amount changing unit includes an exposure time changing unit configured to change exposure time of the photographing optical system, and
the imaging controller is configured to perform, in the exposure amount change control, exposure time change control that controls the exposure time changing unit to sequentially change the exposure time of the photographing optical system to the plurality of different exposure times.

24. The ophthalmic apparatus of any one of claims 1, 7, and 20, wherein
the exposure amount changing unit includes an illumination intensity changing unit configured to change intensity of the illumination light, and
the imaging controller is configured to perform, in the exposure amount change control, illumination intensity change control that controls the illumination intensity changing unit to sequentially change the intensity of the illumination light to a plurality of different intensities.

25. A method for controlling an ophthalmic apparatus configured to image an anterior segment of a subject's eye,
the ophthalmic apparatus comprising:
an illumination optical system configured to project illumination light onto the anterior segment;
a photographing optical system configured to perform imaging of the anterior segment, from a front side, onto which the illumination light is projected;
a focus position changing unit configured to change a focus position of the photographing optical system;
an exposure amount changing unit configured to change an exposure amount for imaging of the anterior segment; and
a processor,
the method comprising:
an imaging step of causing the processor to perform combination control of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire, for each of the plurality of different focus positions, a first image group corresponding to the plurality of different exposure amounts; and
an image processing step of generating a single image by performing compositing on a plurality of first image groups acquired in the imaging step and respectively corresponding to the plurality of different focus positions.

26. A method for controlling an ophthalmic apparatus configured to image an anterior segment of a subject's eye,
the ophthalmic apparatus comprising:
an illumination optical system configured to project illumination light onto the anterior segment;
a photographing optical system configured to perform imaging of the anterior segment, from a front side, onto which the illumination light is projected;
a focus position changing unit configured to change a focus position of the photographing optical system;
an exposure amount changing unit configured to change an exposure amount for imaging of the anterior segment; and
a processor,
the method comprising:
an imaging step of causing the processor to perform combination control of focus position change control that controls the focus position changing unit to sequentially change the focus position of the photographing optical system to a plurality of different focus positions, exposure amount change control that controls the exposure amount changing unit to sequentially change the exposure amount for imaging of the anterior segment to a plurality of different exposure amounts, and imaging control that controls the photographing optical system to acquire time-series images, thereby causing the photographing optical system to acquire a second image group corresponding to a plurality of pairs, each pair including a focus position and an exposure amount and the plurality of pairs including a pair based on a one-to-one correspondence between the plurality of different focus positions and the plurality of different exposure amounts; and
an image processing step of generating a single image by performing compositing on the second image group acquired in the imaging step.

27. A method for controlling an ophthalmic apparatus configured to image an anterior segment of a subject's eye,
the ophthalmic apparatus comprising:
an illumination optical system configured to project illumination light onto the anterior segment;
a photographing optical system configured to perform imaging of the anterior segment, from a front side, onto which the illumination light is projected;
a focus position changing unit configured to change a focus position of the photographing optical system; and
a processor,
the method comprising:
an imaging step of causing the processor to perform combination control of focus position change control that controls the focus position changing unit to sequentially change the focus position of the photographing optical system to a plurality of different focus positions, and imaging control that controls the photographing optical system to acquire time-series images at a constant exposure amount, thereby causing the photographing optical system to acquire a third image group corresponding to the plurality of different focus positions; and
an image processing step of generating a single image by performing compositing on the third image group acquired in the imaging step,
wherein the third image group includes only one image for each of the plurality of different focus positions.

28. A method for controlling an ophthalmic apparatus configured to image an anterior segment of a subject's eye,
the ophthalmic apparatus comprising:
an illumination optical system configured to project illumination light onto the anterior segment;
a photographing optical system configured to perform imaging of the anterior segment, from a front side, onto which the illumination light is projected;
an exposure amount changing unit configured to change an exposure amount for imaging of the anterior segment; and
a processor,
the method comprising:
an imaging step of causing the processor to perform combination control of exposure amount change control that controls the exposure amount changing unit to sequentially change the exposure amount for imaging of the anterior segment to a plurality of different exposure amounts, and imaging control that controls the photographing optical system to acquire time-series images at a constant focus position, thereby causing the photographing optical system to acquire a fourth image group corresponding to the plurality of different exposure amounts; and
an image processing step of generating a single image by performing compositing on the fourth image group acquired in the imaging step,
wherein the fourth image group includes only one image for each of the plurality of different exposure amounts.

29. A computer-readable non-transitory recording medium storing a program for causing a computer to perform the method of any one of claims 25 to 28.
